# EUROPEAN PATENT APPLICATION

(11) **EP 4 434 998 A1**
(43) Date of publication of application: **25.09.2024**
(21) Application number: 22894886.5
(22) Date of filing: 17.11.2022
(51) Int. Cl.: C07K 14/54, C07K 14/715, C07K 16/28, C12N 15/13

(54) **IL-15 MUTANT FUSION PROTEIN PHARMACEUTICAL COMPOSITION**

(30) Priority: 18.11.2021 CN 202111375146; 22.11.2021 CN 202111384988
(71) Applicant: Shandong Simcere Biopharmaceutical Co., Ltd., Shandong 264006 (CN)
(72) Inventor: MA, Fei, Shanghai 201318 (CN); LI, Xinxin, Shanghai 201318 (CN); HU, Jianzhong, Shanghai 201318 (CN); LIU, Yang, Nanjing, Jiangsu 210042 (CN); ZHAO, Xiaofeng, Nanjing, Jiangsu 210042 (CN); CAO, Zhuoxiao, Shanghai 201318 (CN); TANG, Renhong, Shanghai 201318 (CN); REN, Jinsheng, Nanjing, Jiangsu 210042 (CN)
(74) Representative: KATZAROV S.A.
(86) International application number: PCT/CN2022/132510
(87) International publication number: WO 2023/088354

(57) **Abstract**

Disclosed is an IL-15 mutant fusion protein preparation. Specifically, provided is an IL-15 mutant fusion protein pharmaceutical composition. The composition comprises an IL-15 mutant fusion protein, a buffer solution, a protein stabilizer and a surfactant. The pharmaceutical composition can maintain the stability of the IL-15 mutant fusion protein in the long-term storage and transportation process of the product, and thus, the stability, safety and effectiveness of the drug are ensured.

## Description

### CROSS-REFERENCE TO RELATED APPLICATIONS

The present application claims the benefit and priority to the following 2 Chinese Patent Applications for Invention, the contents of which are incorporated herein by reference in their entirety: Chinese Patent Application No. 202111375146.0 filed with China National Intellectual Property Administration on Nov. 18, 2021, and Chinese Patent Application No. 202111384988.2 filed with China National Intellectual Property Administration on Nov. 22, 2021.

### TECHNICAL FIELD

The present invention relates to the field of pharmaceutical formulations, in particular to a pharmaceutical composition comprising an IL-15 variant fusion protein.

### BACKGROUND

Formulations of macromolecular protein drugs (such as antibodies) are usually injections, and are suitable for parenteral administration. The administration mode usually includes subcutaneous, intravenous, and intramuscular administration, and the like. Protein is unstable in solutions, and particles, aggregation, and the like are very easily formed. Stability has become a problem in the development of macromolecular protein drugs. Therefore, the development of formulations of macromolecular protein drugs (such as antibodies) first needs to solve the stability problem of protein.

### SUMMARY

The present invention provides a liquid formulation and a freeze-dried formulation of an IL-15 variant fusion protein. The formulation can maintain the stability of the IL-15 variant fusion protein in long-term storage and transportation processes of the product, thereby ensuring the stability, safety, and effectiveness of the drug.

In a first aspect, the present disclosure provides a pharmaceutical composition, wherein the pharmaceutical composition comprises an IL-15 variant fusion protein, a buffer, a protein stabilizer, and a surfactant.

In one embodiment, the IL-15 variant fusion protein comprises an IL-15 variant, an immunoglobulin molecule fused to the IL-15 variant or a portion thereof, and/or IL-15Rα.

In one embodiment, the IL-15 variant comprises a mutation at one or more amino acid residues corresponding to Asp8, Ile6, Val3, and His105 of wild-type IL-15.

In one embodiment, the mutation is substitution, insertion, or deletion.

In a specific embodiment, the mutation is an amino acid substitution selected from the group consisting of: Asp8Ser (D8S), Asp8Gly (D8G), Asp8 Glu (D8E), Val3Leu (V3L), Ile6Asp (I6D), and/or His105Lys (H105K).

In a specific embodiment, the IL-15 variant comprises the following mutation or combination of mutations: (1) Asp8Ser; (2) Asp8Gly; (3) His105 Lys; (4) Asp8Glu and Val3Leu; (5) Val3Leu and Ile6Asp; or (6) Asp8Ser and His105Lys.

In a specific embodiment, the amino acid sequence of the IL-15 variant is set forth in SEQ ID NO: 2, SEQ ID NO: 3, SEQ ID NO: 4, SEQ ID NO: 5, SEQ ID NO: 6, or SEQ ID NO: 7; the amino acid sequence of the wild-type IL-15 is set forth in SEQ ID NO: 1.

In another embodiment, the order in which the domains in the IL-15 variant fusion protein are linked from the N-terminus to the C-terminus is:
(1) an immunoglobulin molecule or a portion thereof, IL-15Rα, and an IL-15 variant; (2) an immunoglobulin molecule or a portion thereof, an IL-15 variant, and IL-15Rα; (3) an IL-15 variant, IL-15Rα, and an immunoglobulin molecule or a portion thereof; (4) IL-15Rα, an IL-15 variant, and an immunoglobulin molecule or a portion thereof; (5) an IL-15 variant and an immunoglobulin molecule or a portion thereof; (6) an immunoglobulin molecule or a portion thereof and IL-15Rα; (7) IL-15Rα and an immunoglobulin molecule or a portion thereof; (8) an IL-15 variant and IL-15Rα; or (9) IL-15Rα and an IL-15 variant;
preferably, when the IL-15Rα or the IL-15 variant is fused to the immunoglobulin molecule, they are fused at the N-terminus of the heavy chain variable region of the immunoglobulin molecule or the C-terminus of the immunoglobulin Fc region; when the IL-15Rα or the IL-15 variant is fused to the immunoglobulin Fc region, they are fused at the N-terminus or C-terminus of the immunoglobulin Fc region.

In another embodiment, the IL-15 variant is fused to the immunoglobulin molecule or the portion thereof with or without a linker peptide, or the IL-15 variant is fused to IL-15Rα with or without a linker peptide; preferably, a linker peptide is used; preferably, the linker peptide set forth in SEQ ID NO: 13, SEQ ID NO: 14, SEQ ID NO: 15, or SEQ ID NO: 16 is used;
preferably, the IL-15 variant is fused to IL-15Rα with or without a linker peptide, and is then fused to the immunoglobulin molecule or the portion thereof; preferably, a linker peptide is used; preferably, the linker peptide set forth in SEQ ID NO: 13, SEQ ID NO: 15, or SEQ ID NO: 16 is used.

In a specific embodiment, the immunoglobulin molecule is an anti-PD-L1 antibody or antigen-binding fragment; the immunoglobulin molecule portion is the immunoglobulin Fc region; preferably, the anti-PD-L1 antibody or antigen-binding fragment is selected from Tecentriq, KN-035, or 794-h1-71;
preferably, the heavy chain of the anti-PD-L1 antibody has the sequence set forth in SEQ ID NO: 19 or SEQ ID NO: 20, and the light chain has the sequence set forth in SEQ ID NO: 21;
preferably, the immunoglobulin Fc region is selected from the Fc region of any one of IgG1, IgG2, IgG3, IgG4, IgA, IgM, IgE, or IgD, preferably comprising the sequence of the constant region of human or murine antibody IgG1, IgG2, IgG3, or IgG4; preferably, the amino acid sequence of the immunoglobulin Fc region is set forth in SEQ ID NO: 12.

In a specific embodiment, the IL-15 variant fusion protein comprises:
(1) monomer one independently having the sequence set forth in SEQ ID NO: 22, 23, 24, 25, or 26, and comprising the heavy chain of the anti-PD-L1 antibody; the light chain of the anti-PD-L1 antibody having the sequence set forth in SEQ ID NO: 21;
(2) an amino acid sequence having at least 90% identity, preferably at least 91%, 92%, 93%, 94%, 95%, 96%, 97%, 98%, or 99% identity, to the sequence set forth in (1) above.

In another embodiment, the concentration of the IL-15 variant fusion protein is 1 mg/mL to 100 mg/mL, preferably 25-95 mg/mL, and more preferably 50 mg/mL or 25 mg/mL.

In another embodiment, the buffer is selected from an acetic acid-sodium acetate buffer, a citric acid-sodium citrate buffer, a histidine-histidine hydrochloride buffer, and a disodium hydrogen phosphate-sodium dihydrogen phosphate buffer, preferably an acetic acid-sodium acetate buffer or a histidine-histidine hydrochloride buffer.

In a specific embodiment, the concentration of the buffer is 10-100 mM, preferably 15-30 mM, and more preferably 20 mM.

In another embodiment, the protein stabilizer is selected from sodium chloride, glycine, arginine hydrochloride, sucrose, trehalose, mannitol, and sorbitol, preferably sucrose and trehalose.

In a specific embodiment, the concentration of the protein stabilizer is 1-10% (w/v), preferably 2% (w/v), 4.5% (w/v), or 8% (w/v), and more preferably 8% (w/v).

In another embodiment, the surfactant is selected from polysorbate, poloxamer, and glycerol fatty acid ester, preferably polysorbate 80 or poloxamer 188.

In a specific embodiment, the concentration of the surfactant is 0.01-0.1% (w/v), preferably 0.02% (w/v), 0.04% (w/v), 0.06% (w/v), or 0.08% (w/v).

In another embodiment, the pH of the pharmaceutical composition is about 4.5-7.5, preferably 4.5-6.5, and more preferably 4.5, 5, and 6.

In a second aspect, the present disclosure provides a pharmaceutical composition, wherein the pharmaceutical composition comprises:
a) an IL-15 variant fusion protein at a concentration of 1 mg/mL to 100 mg/mL, b) a histidine-histidine hydrochloride buffer at a concentration of 10-100 mM, pH 4.5-7.5, c) trehalose at a concentration of 1-10% (w/v), and d) poloxamer at a concentration of 0.01-0.1% (w/v);
preferably, the pharmaceutical composition comprises: a) an IL-15 variant fusion protein at a concentration of 25 mg/mL to 95 mg/mL, b) a histidine-histidine hydrochloride buffer at a concentration of 15-30 mM, pH 5.5-6.5, c) trehalose at a concentration of 2-8% (w/v), and d) poloxamer 188 at a concentration of 0.04% (w/v);
preferably, the pharmaceutical composition is in a liquid form or a freeze-dried powder formulation. In a third aspect, the present disclosure provides a method for preparing a pharmaceutical composition, wherein the method comprises the step of performing buffer exchange on an IL-15 variant fusion protein solution, the buffer is preferably a histidine-histidine hydrochloride buffer, the concentration of the buffer is preferably 20 mM, and the pH of the buffer is preferably 6;
preferably, the preparation method further comprises the step of freeze-drying;
more preferably, the freeze-drying step comprises: (1) cooling the drying box of a vacuum freeze dryer to -45 °C and keeping for 3-5 h; (2) heating the drying box to -5 °C and keeping for 3-5 h; (3) cooling the drying box to -45 °C and keeping for 3-5 h (in the pre-freezing process of steps (1) - (3), the heating rate and the cooling rate are kept at 0.5-1 °C/min); (4) the degree of vacuum in primary drying is 0.1 mBar, the temperature is kept at -30 °C to 20 °C, and the total time of the step should be greater than 30 h; (5) after primary drying, heating to 25 °C at a heating rate of 0.1-0.3 °C/min, starting desorption drying, keeping the degree of vacuum at 0.1 mBar for 1-2 h, then creating ultimate vacuum, and keeping the temperature unchanged for longer than 15 h.

In a fourth aspect, the present disclosure provides a pharmaceutical composition comprising an IL-15 variant fusion protein, wherein the pharmaceutical composition is prepared by the method according to the third aspect.

In a fifth aspect, the present disclosure provides a freeze-dried formulation comprising an IL-15 variant fusion protein, wherein the formulation is obtained by freeze-drying of the pharmaceutical composition according to the first to second aspects.

In a sixth aspect, the present disclosure provides a reconstituted solution comprising an IL-15 variant fusion protein, wherein the reconstituted solution is prepared by reconstituting the freeze-dried formulation according to the fifth aspect.

In a seventh aspect, the present disclosure provides use of the pharmaceutical composition according to the first to second aspects, a product prepared by the method according to the third aspect, the pharmaceutical composition according to the fourth aspect, the freeze-dried formulation according to the fifth aspect, or the reconstituted solution according to the sixth aspect in preparing a drug for preventing and/or treating a disease in an individual, wherein the disease is preferably a tumor or an inflammatory disease; preferably, the tumor is selected from glioblastoma, prostate cancer, hematologic cancer, B-cell tumor, multiple myeloma, B-cell lymphoma, B-cell non-Hodgkin's lymphoma, Hodgkin's lymphoma, chronic lymphocytic leukemia, acute myelogenous leukemia, cutaneous T-cell lymphoma, T-cell lymphoma, solid tumor, urothelial/bladder cancer, melanoma, lung cancer, renal cell carcinoma, breast cancer, gastric and esophageal cancer, prostate cancer, pancreatic cancer, colorectal cancer, ovarian cancer, non-small cell lung cancer, and head and neck squamous cell carcinoma.

In an eighth aspect, the present disclosure provides a method for preventing and/or treating a disease in an individual, wherein the method comprises administering to a patient in need thereof the pharmaceutical composition according to the first to second aspects, a product prepared by the method according to the third aspect, the pharmaceutical composition according to the fourth aspect, the freeze-dried formulation according to the fifth aspect, or the reconstituted solution according to the sixth aspect, wherein the disease is preferably a tumor or an inflammatory disease; preferably, the tumor is selected from glioblastoma, prostate cancer, hematologic cancer, B-cell tumor, multiple myeloma, B-cell lymphoma, B-cell non-Hodgkin's lymphoma, Hodgkin's lymphoma, chronic lymphocytic leukemia, acute myelogenous leukemia, cutaneous T-cell lymphoma, T-cell lymphoma, solid tumor, urothelial/bladder cancer, melanoma, lung cancer, renal cell carcinoma, breast cancer, gastric and esophageal cancer, prostate cancer, pancreatic cancer, colorectal cancer, ovarian cancer, non-small cell lung cancer, and head and neck squamous cell carcinoma.

### TERMINOLOGY AND DEFINITIONS

Unless otherwise stated, the terms used herein have the meanings that are commonly understood by those of ordinary skill in the art. For a term explicitly defined herein, the meaning of the term shall be subject to the definition.

As used herein, the term "antibody" (Ab) refers to an immunoglobulin molecule that specifically binds to a target antigen or has immunoreactivity, including polyclonal, monoclonal, genetically engineered and other modified forms of antibodies (including, but not limited to, chimeric antibodies, humanized antibodies, fully human antibodies, heteroconjugated antibodies (e.g., bispecific, trispecific and tetraspecific antibodies, diabodies, triabodies, and tetrabodies), and antibody conjugates), and antigen-binding fragments of antibodies (including, for example, Fab', F(ab')2, Fab, Fv, rIgG, and scFv fragments). Furthermore, unless otherwise indicated, the term "monoclonal antibody" (mAb) is intended to include intact antibody molecules and incomplete antibody fragments (e.g., Fab and F(ab')2 fragments which are short of the Fc fragment of an intact antibody (cleared more rapidly from the animal circulation) and thus are short of Fc-mediated effector function (see Wahl et al., J.Nucl.Med. 24: 316, 1983, the content of which is incorporated herein by reference) capable of specifically binding to a target protein).

As used herein, the term "IL-15" or "IL15" (interleukin-15) is a pleiotropic cytokine with the function of activating T cells, B cells, and NK cells and mediating the proliferation and survival of the cells. Furthermore, IL-15 is capable of activating, maintaining and amplifying CD8⁺ memory T cells. The "IL-15" or "IL-15 peptide fragment" or "IL-15 polypeptide" according to the present invention may be any IL-15 (interleukin 15) or a variant thereof, such as human IL-15 or non-human mammalian IL-15 or non-mammalian IL-15. Exemplary non-human mammals are, for example, pigs, rabbits, monkeys, chimpanzees, mice, etc., and non-mammals are, for example, chickens, etc. Preferably the human interleukin 15 mature molecule, see database UniProtKB, accession No. P40933, 49-162aa.

As used herein, the term "IL-15 wild type" or "wild-type IL-15" refers to a naturally derived human IL-15 or non-human mammalian IL-15 or non-mammalian IL-15; it may also refer to an IL-15 polypeptide which is already common in the art, the wild type also being denoted WT or wt. Preferably, the amino acid sequence of "IL-15 wild type" or "wild-type IL-15" is (SEQ ID NO: 1) NWVNVISDLKKIEDLIQSMHIDATLYTESDVHPSCKVTAMKCFLLELQVISLESGDASIHDTV ENLIILANNSLSSNGNVTESGCKECEELEEKNIKEFLQSFVHIVQMFINTS.

As used herein, the term "IL-15 variant" refers to a variant molecule obtained through one or more amino acid substitution, addition, or deletion mutations, which increases or decreases affinity between IL-15 and its receptor, or enhances or reduces activity in stimulating proliferation activity and cytokine release of specific cell lines, T cells, or NK cells.

In the present disclosure, in one of the preferred examples, the "IL-15 variant" independently has the amino acid sequence set forth below:
IL-15 variant #1 (D8S) amino acid sequence (SEQ ID NO: 2): 
NWVNVISSLKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTS;
IL-15 variant #2 (H105K) amino acid sequence (SEQ ID NO: 3): 
NWVNVISDLKKIEDLIQSMHIDATLYTESDVHPSCKVTAMKCFLLELQVISLESGDASIHDTV
ENLIILANNSLSSNGNVTESGCKECEELEEKNIKEFLQSFVKIVQMFINTS;
IL-15 variant #3 (D8G) amino acid sequence (SEQ ID NO: 4): 
NWVNVISGLKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTS;
IL-15 variant #6 (D8S/H105K) amino acid sequence (SEQ ID NO: 5): 
NWVNVISSLKKIEDLIQSMHIDATLYTESDVHPSCKVTAMKCFLLELQVISLESGDASIHDTV
ENLIILANNSLSSNGNVTESGCKECEELEEKNIKEFLQSFVKIVQMFINTS;
IL-15 variant #7 (D8E/V3L) amino acid sequence (SEQ ID NO: 6): 
NWLNVISELKKIEDLIQSMHIDATLYTESDVHPSCKVTAMKCFLLELQVISLESGDASIHDTV
ENLIILANNSLSSNGNVTESGCKECEELEEKNIKEFLQSFVHIVQMFINTS;
IL-15 variant #8 (V3L/I6D) amino acid sequence (SEQ ID NO: 7):

NWLNVDSDLKKIEDLIQSMHIDATLYTESDVHPSCKVTAMKCFLLELQVISLESGDASIHDT
VENLIILANNSLSSNGNVTESGCKECEELEEKNIKEFLQSFVHIVQMFINTS.

As used herein, the term "IL-15Rα" may be IL-15Rα of any species or a functional fragment thereof, such as human IL-15Rα or non-human mammalian IL-15Rα or non-mammalian IL-15Rα. Exemplary non-human mammals are, for example, pigs, rabbits, monkeys, chimpanzees, mice, etc., and non-mammals are, for example, chickens, etc. Preferably human IL-15Rα; preferably a human interleukin 15 receptor α extracellular domain fragment, abbreviated as IL-15Rα ECD.

The term "IL-15Rα variant" in the present invention refers to a functional variant formed by one or more amino acid deletion, insertion, or substitution mutations in IL-15Rα and having the ability to bind to its ligand molecule, such as IL15, preferably a human IL15Rα molecule, and more preferably a shortened form of a human IL-15Rα extracellular domain fragment, i.e. a molecule having the activity of the human interleukin 15 receptor α obtained by one or more amino acid deletion mutations starting from the C-terminus of the extracellular domain fragment, preferably a deletion mutation form retaining 65-120 amino acids, and more preferably a deletion mutation shortened form retaining 65-102 amino acids, such as IL-15Rα-sushi; preferably, the amino acid sequences of IL-15Rα-sushi are respectively
IL-15Rα-sushi-1 (SEQ ID NO.8): 
CPPPMSVEHADIWVKSYSLYSRERYICNSGFKRKAGTSSLTECVLNKATNVAHWTTPSLKCI
RDPALVHQRPAPP.
IL-15Rα-sushi-2: (SEQ ID NO.9): 
CPPPMSVEHADIWVKSYSLYSRERYICNSGFKRKAGTSSLTECVLNKATNVAHWTTPSLKCI
R.
IL-15Rα-sushi-3: (SEQ ID NO.10): 
ITCPPPMSVEHADIWVKSYSLYSRERYICNSGFKRKAGTSSLTECVLNKATNVAHWTTPSLK
CIRDPALVHQRPAPP.
IL-15Rα-sushi-4: (SEQ ID NO.11):

ITCPPPMSVEHADIWVKSYSLYSRERYICNSGFKRKAGTSSLTECVLNKATNVAHWTTPSLK
CIR.

The term "immunoglobulin Fc region" as used herein refers to the constant region of an immunoglobulin chain, particularly the carboxy-terminus of an immunoglobulin heavy chain constant region or a portion thereof, which has no antigen-binding activity and is the site of interaction of an antibody molecule and an effector molecule with a cell. The "immunoglobulin Fc region" of the present invention may be any Fc or a variant thereof, derived from a human or non-human mammal. For example, the immunoglobulin Fc region may comprise a combination of two or more of the heavy chain CH1, CH2, CH3, and CH4 domains with an immunoglobulin hinge region. The Fc may be derived from a different species, preferably a human immunoglobulin. Based on the amino acid sequence of the heavy chain constant region, immunoglobulins can be divided into different types. There are mainly 5 types of immunoglobulins: IgA, IgD, IgE, IgG, and IgM. Some of them can be further divided into subtypes (isoforms), such as IgG-1, IgG-2, IgG-3, IgG-4, IgA-1, and IgA-2. The "Fc region" preferably comprises at least one immunoglobulin hinge region, and CH2 and CH3 domains of IgG. More preferably, it comprises one CH2 domain, one CH3 domain, and one immunoglobulin hinge region of IgG1. The starting amino acid position of the hinge region may vary.

In the present disclosure, in one of the preferred examples, the "immunoglobulin Fc region" has the amino acid sequence set forth below:

SEQ ID NO.12:
EPKSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK.

As used herein, the term "peptide linker/linker peptide (linker)" refers to a peptide used in the present invention to link IL-15 to another protein molecule or protein fragment to ensure proper folding and stability of protein. The another molecule includes, but is not limited to, IL-15Rα, Fc, Fc variants, antibodies, and the like. The "linker peptide" of the present invention is preferably (GGGGS)n (SEQ ID NO.27-31), wherein n may be 0, 1, 2, 3, 4, 5 or more, preferably 2-4, or preferably SGGSGGGGSGGGSGGGGSLQ (SEQ ID NO. 13). If the linker peptide sequence is too short, it may affect the folding of the higher-order structures of the two proteins, causing them to interfere with each other; if the linker peptide sequence is too long, immunogenicity problems are involved, since the linker peptide sequence itself is a new antigen.

In the present disclosure, in one of the preferred examples, the "peptide linker/linker peptide (linker)" independently has the amino acid sequence set forth below:
SEQ ID NO. 13: SGGSGGGGSGGGSGGGGSLQ;
SEQ ID NO. 14: GGGGSGGGGSGGGGS ;
SEQ ID NO. 15: EF;
SEQ ID NO. 16: SGGGSGGGGSGGGGSGGGGSGGGSLQ.

In the present disclosure, in the preferred examples, the preferred antibody heavy chain and antibody light chain are derived from an anti-PD-1 antibody or an anti-PD-L1 antibody, more preferably derived from a humanized anti-PD-1 antibody or anti-PD-L1 antibody; as used herein, the term "PD-L1" refers to programmed death ligand-1, also known as CD279 (cluster of differentiation 279), an important immunosuppressive molecule, and the PD-L1 is preferably human PD-L1.

In the present disclosure, in one of the preferred examples, the "anti-PD-L1 antibody" independently has the amino acid sequence set forth below:
Heavy chain of PD-L1-1 SEQ ID NO: 17: 
EVQLVESGGGLVQPGGSLRLSCAASGFTFSDSWIHWVRQAPGKGLEWVAWISPYGGSTYY
ADSVKGRFTISADTSKNTAYLQMNSLRAEDTAVYYCARRHWPGGFDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK;
Light chain of PD-L1-1 SEQ ID NO: 18: DIQMTQSPSSLSASVGDRVTITCRASQDVSTAVAWYQQKPGKAPKLLIYSASFLYSGVPSRFS 
GSGSGTDFTLTISSLQPEDFATYYCQQYLYHPATFGQGTKVEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC;
Heavy chain of PD-L1-2-1 SEQ ID NO: 19: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGK;
Heavy chain of PD-L1-2-2 SEQ ID NO: 20: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPEAAGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGK;
Light chain of PD-L1-2-1 or PD-L1-2-2 SEQ ID NO: 21:

EIVMTQSPPTLSLSPGERVTLSCKSSQSLLYSSNQKNSLAWYQQKPGQAPRLLIYWASTRES
GIPARFSGSGSGTDFTLTISSLQPEDFAVYYCQQYYGYPYTFGQGTKLEIKRTVAAPSVFIFPP
SDEQLKSGTASVVCLLNNFYPREAKVQWKVDNALQSGNSQESVTEQDSKDSTYSLSSTLTL
SKADYEKHKVYACEVTHQGLSSPVTKSFNRGEC.

As used herein, the term "fusion protein" refers to a protein product obtained by gene recombination, which is expressed under the control of the same regulatory sequence by linking the coding regions of two or more genes by gene recombination method, chemical method, or other appropriate methods. In the fusion protein of the present invention, the coding regions of two or more genes may be fused at one or several positions by the sequence encoding a peptide linker or linker peptide. Peptide linkers or linker peptides may also be used to construct the fusion protein of the present invention. The term "fusion protein" of the present invention further includes an antibody/Fc fusion protein construct/complex, or a composition of an antibody/Fc fusion protein construct/complex formed by non-covalent means. For example, the fusion protein of the present invention may be shown as the following structure:
(1) an IL-15 fusion protein that is a homodimer comprising two monomers; the monomer comprises an antibody heavy chain, an antibody light chain, IL-15, and IL-15Rα sushi; for example, the antibody heavy chain Fc is fused to the IL-15Rα sushi and co-expressed with the antibody light chain and IL-15-WT (wild type) or an IL-15 variant, such that IL-15 non-covalently links to IL-15Rα sushi;
(2) an IL-15 fusion protein that is a homodimer comprising two monomers; the monomer comprises an antibody heavy chain, an antibody light chain, IL-15, and IL-15Rα sushi; for example, three parts of the antibody heavy chain Fc, the IL-15Rα sushi, and IL-15-WT or an IL-15 variant are linked in series, fused and expressed by a linker, and are co-expressed with the antibody light chain;
(3) an IL-15 fusion protein that is a homodimer comprising two monomers; the monomer comprises Fc, IL-15, and IL-15Rα sushi; for example, IL-15-WT or an IL-15 variant is linked to the IL15-Rαsushi by a linker, and IL15-Rαsushi is linked to Fc by a linker; or
(4) an IL-15 fusion protein that is a homodimer comprising two monomers; the monomer comprises Fc, IL-15, and IL-15Rα sushi; for example, IL15-Rαsushi is linked to IL-15-WT or an IL-15 variant by a linker, and IL-15 is linked to Fc by a linker.

In the present disclosure, the term "monomer one" refers to a protein product obtained by gene recombination, which is expressed under the control of the same regulatory sequence by linking the coding regions of a plurality of genes as follows by gene recombination method, chemical method, or other appropriate methods: (1) a PD-L1 antibody heavy chain; (2) a linker linking heavy chain Fc and IL-15Rα sushi; (3) IL-15Rα sushi; (4) a linker linking IL-15Rα sushi and IL-15 (WT or variant); (4) IL-15 (WT or variant).

In the present disclosure, in one of the preferred examples, the "IL-15 fusion protein" independently has the amino acid sequence set forth below:
#6-1-1, monomer one: SEQ ID NO: 22: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGSCPPPMSVEHADIWVKSYSLYSRERYICNSGFKRKAGTSSLTEC
VLNKATNVAHWTTPSLKCIRDPALVHQRPAPPSGGSGGGGSGGGSGGGGSLQNWVNVISSL
KKIEDLIQSMHIDATLYTESDVHPSCKVTAMKCFLLELQVISLESGDASIHDTVENLIILANNS
LSSNGNVTESGCKECEELEEKNIKEFLQSFVKIVQMFINTS;
#6-1-1, light chain: SEQ ID NO: 21;
#6-1-2, monomer one, SEQ ID NO: 23: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPEAAGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGSCPPPMSVEHADIWVKSYSLYSRERYICNSGFKRKAGTSSLTEC
VLNKATNVAHWTTPSLKCIRDPALVHQRPAPPSGGSGGGGSGGGSGGGGSLQNWVNVISSL
 
KKIEDLIQSMHIDATLYTESDVHPSCKVTAMKCFLLELQVISLESGDASIHDTVENLIILANNS
LSSNGNVTESGCKECEELEEKNIKEFLQSFVKIVQMFINTS;
#6-1-2, light chain: SEQ ID NO: 21;
#7-1, monomer one, SEQ ID NO: 24: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGSCPPPMSVEHADIWVKSYSLYSRERYICNSGFKRKAGTSSLTEC
VLNKATNVAHWTTPSLKCIRDPALVHQRPAPPSGGSGGGGSGGGSGGGGSLQNWLNVISEL
KKIEDLIQSMHIDATLYTESDVHPSCKVTAMKCFLLELQVISLESGDASIHDTVENLIILANNS
LSSNGNVTESGCKECEELEEKNIKEFLQSFVHIVQMFINTS;
#7-1, light chain: SEQ ID NO: 21;
#8-1, monomer one, SEQ ID NO: 25: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGSCPPPMSVEHADIWVKSYSLYSRERYICNSGFKRKAGTSSLTEC
VLNKATNVAHWTTPSLKCIRDPALVHQRPAPPSGGSGGGGSGGGSGGGGSLQNWLNVDSD
LKKIEDLIQSMHIDATLYTESDVHPSCKVTAMKCFLLELQVISLESGDASIHDTVENLIILANN
SLSSNGNVTESGCKECEELEEKNIKEFLQSFVHIVQMFINTS;
#8-1, light chain: SEQ ID NO: 21;
#9-1, monomer one, SEQ ID NO: 26: 
QVQLQQSGPGLVKPSQTLSLTCAVSGDSITSGYWNWIRKFPSRGLEYMGYISYSGSTYYNPF
LKSRISINRDTSKNQYYLQLNSVTPEDTAVYYCAKMGDWLAWFAYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVM
HEALHNHYTQKSLSLSPGSCPPPMSVEHADIWVKSYSLYSRERYICNSGFKRKAGTSSLTEC
VLNKATNVAHWTTPSLKCIRDPALVHQRPAPPSGGSGGGGSGGGSGGGGSLQNWVNVISDL
KKIEDLIQSMHIDATLYTESDVHPSCKVTAMKCFLLELQVISLESGDASIHDTVENLIILANNS
LSSNGNVTESGCKECEELEEKNIKEFLQSFVHIVQMFINTS;
#9-1, light chain: SEQ ID NO: 21.

As used herein, the term "percent (%) sequence identity" refers to the percentage of identity between amino acid (or nucleotide) residues of a candidate sequence and amino acid (or nucleotide) residues of a reference sequence after aligning the sequences and introducing gaps, if necessary, to achieve maximum percent sequence identity (e.g., gaps may be introduced in one or both of the candidate sequence and the reference sequence for optimal alignment, and non-homologous sequences may be omitted for the purpose of comparison). Alignment may be carried out in a variety of ways well known to those skilled in the art for the purpose of determining percent sequence identity, for example, using publicly available computer software such as BLAST, ALIGN, or Megalign (DNASTAIi) software. Those skilled in the art can determine appropriate parameters for measuring alignment, including any algorithms required to achieve maximum alignment of the full length of the aligned sequences. For example, a reference sequence aligned for comparison with a candidate sequence may show that the candidate sequence exhibits 50% to 100% sequence identity over the full length of the candidate sequence or a selected portion of contiguous amino acid (or nucleotide) residues of the candidate sequence. The length of the candidate sequence aligned for the purpose of comparison may be, e.g., at least 30% (e.g., 30%, 40%, 50%, 60%, 70%, 80%, 90%, or 100%) of the length of the reference sequence. When a position in the candidate sequence is occupied by the same amino acid (or nucleotide) residue at the corresponding position in the reference sequence, then the molecules are identical at that position.

As used herein, "pharmaceutical composition" refers to a mixture containing one or more of the compounds or the physiologically/pharmaceutically acceptable salts or prodrugs thereof described herein and other chemical components such as physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity. As used herein, "pharmaceutical composition" and "formulation" are not mutually exclusive.

As used herein, "freeze-dried formulation" refers to a formulation or a pharmaceutical composition obtained by freeze-drying of a pharmaceutical composition or a formulation in liquid or solution form in vacuum.

### BRIEF DESCRIPTION OF THE DRAWING

FIG. 1 shows a schematic diagram of the structure of IL-15 fusion protein.
FIG. 2 shows the trend of the investigated SE-HPLC result summary with samples placed at 40 °C in the stability test.
FIG. 3 shows the trend of the investigated CE-SDS (NR) result summary with samples placed at 40 °C in the stability test.
FIG. 4 shows the trend of the investigated CE-SDS (R) result summary with samples placed at 40 °C in the stability test.
FIG. 5 shows the trend of the investigated iCIEF result summary with samples placed at 40 °C in the stability test.
FIG. 6 shows the effect of the test drug on T cell proliferation and IFN-γ secretion determined by mixed lymphocyte reaction (MLR).
FIG. 7A shows FACS of human myeloid-derived suppressor cell (MDSC) proliferation; B shows the effect of the test drug on human myeloid-derived suppressor cell proliferation determined by FACS; C shows the absolute cell count results of MDSCs.
FIG. 8A shows the NPG mouse tumor growth curve; B shows the single mouse growth curve comparison of the Tecentriq group and the #6-1 group; C shows the single mouse growth curve comparison of the aHel-IL15 group and the #6-1 group.
FIG. 9 shows the NPG mouse body weight growth curve.
FIG. 10 shows the results of plasma concentration in NPG mouse tumor determined by Elisa.
FIG. 11 shows the results of the binding of the test drug to tumor tissue cells determined by FACS.

### DETAILED DESCRIPTION

The present invention will be further described with reference to specific examples, and the advantages and features of the present invention will become more apparent with the description. Experimental procedures without specified conditions in the examples are conducted according to conventional conditions or conditions recommended by the manufacturers. Reagents or instruments without specified manufacturers used herein are conventional products that are commercially available.

The examples are exemplary only and do not limit the scope of the present invention in any way. It will be understood by those skilled in the art that various changes or substitutions in form and details may be made to the technical solutions of the present invention without departing from the spirit and scope of the present invention, and that these changes and substitutions shall fall within the scope of the present invention.

The present invention is described in more detail by the following examples, but the scope of the present invention is not limited thereto.

### Example 1. Design, Expression, and Purification of IL-15 Fusion Protein

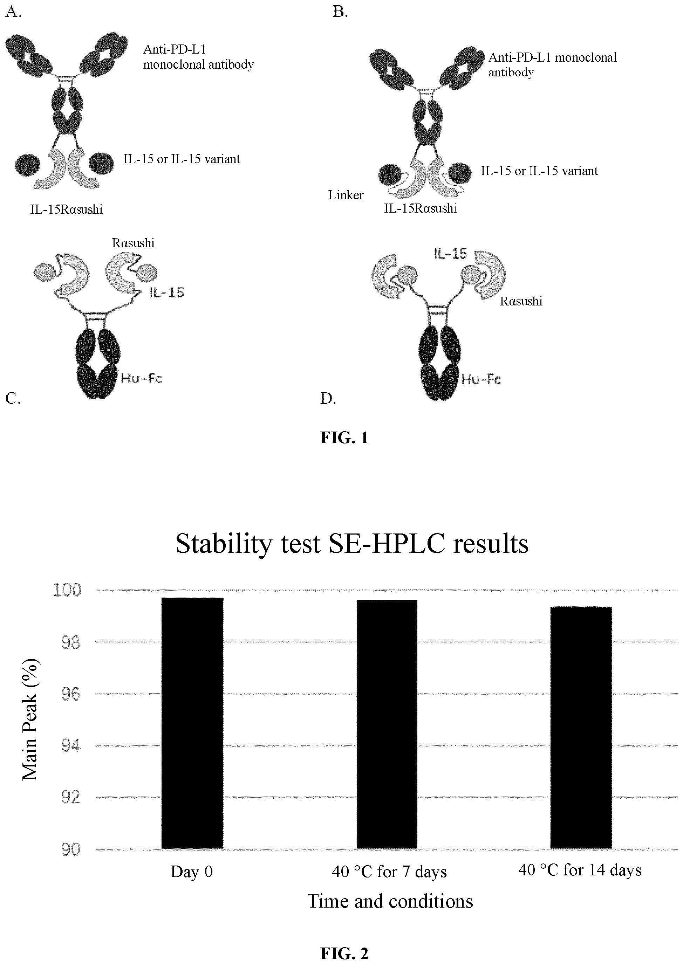

1.1 Structural design (format) of IL-15 fusion protein (antibody fusion construct) has four modes:
format 1: as the structure shown in A in FIG. 1, an IL-15 fusion protein that is a homodimer comprising two monomers; the monomer comprises an antibody heavy chain, an antibody light chain, IL-15, and IL-15Rα sushi; the antibody heavy chain Fc end is fused to the IL-15Rα sushi and co-expressed with the monoclonal antibody light chain and IL-15-WT (wild type) or an IL-15 variant, such that IL-15 non-covalently links to IL-15Rα sushi;
format 2: as the structure shown in B in FIG. 1, an IL-15 fusion protein that is a homodimer comprising two monomers; the monomer comprises an antibody heavy chain, an antibody light chain, IL-15, and IL-15Rα sushi; three parts of the antibody heavy chain Fc end, the IL-15Rα sushi, and IL-15-WT or an IL-15 variant are linked in sequence in series, fused and expressed by a linker, and are co-expressed with the antibody light chain;
format 3: as the structure shown in C in FIG. 1, an IL-15 fusion protein that is a homodimer comprising two monomers; the monomer comprises Fc, IL-15, and IL-15Rα sushi; IL-15-WT or an IL-15 variant is linked to the IL15-Rαsushi by a linker, and IL15-Rαsushi is linked to Fc by a linker; or
format 4: as the structure shown in D in FIG. 1, an IL-15 fusion protein that is a homodimer comprising two monomers; the monomer comprises Fc, IL-15, and IL-15Rα sushi; IL15-Rαsushi is linked to IL-15-WT or an IL-15 variant by a linker, and IL-15 is linked to Fc by a linker.

For the details of design of various IL-15 fusion proteins, see Table 1 and Table 2.

**Table 1. Component of anti-PD-L1-IL-15 fusion protein molecule**

| **Domains of anti-PD-L1-IL-15 fusion protein molecule and corresponding amino acid sequences** | | | | | | |
|---|---|---|---|---|---|---|
| **Fusion protein No.** | **PD-L1 monoclonal antibody heavy chain sequence** | **IL15Rα sushi** | **Linker** | **IL-15 protein** | **PD-L1 monoclonal antibody light chain sequence** | **Fusion protein structure fromat** |
| #1-1 | SEQ ID NO. 17 | SEQ ID NO.8 | NA | SEQ ID NO.2 | SEQ ID NO. 18 | format 1 |
| #2-1 | SEQ ID NO. 17 | SEQ ID NO.8 | NA | SEQ ID NO.3 | SEQ ID NO. 18 | format 1 |
| #6-1-1 | SEQ ID NO. 19 | SEQ ID NO.8 | SEQ ID NO. 13 | SEQ ID NO. 5 | SEQ ID NO.21 | format 2 |
| #6-1-2 | SEQ ID NO.20 | SEQ ID NO.8 | SEQ ID NO. 13 | SEQ ID NO. 5 | SEQ ID NO.21 | format 2 |
| #7-1 | SEQ ID NO.20 | SEQ ID NO.8 | SEQ ID NO. 13 | SEQ ID NO.6 | SEQ ID NO.21 | format 2 |
| #8-1 | SEQ ID NO.20 | SEQ ID NO.8 | SEQ ID NO. 13 | SEQ ID NO.7 | SEQ ID NO.21 | format 2 |
| #9-1 | SEQ ID NO.20 | SEQ ID NO.8 | SEQ ID NO. 13 | SEQ ID NO.1 | SEQ ID NO.21 | format 2 |

**Table 2. Component of IL-15-Fc fusion protein molecule**

| **Domains of IL-15-Fc fusion protein molecule and corresponding amino acid sequences** | | | | | | |
|---|---|---|---|---|---|---|
| **Fusion protein No.** | **IL-15 protein** | **Linker1** | **IL15Rα sushi** | **Linker2** | **Human Fc** | **Fusion protein structure fromat** |
| #3-1 | SEQ ID NO.4 | SEQ ID NO. 13 | SEQ ID NO.10 | SEQ ID NO. 14 | SEQ ID NO. 12 | format 4 |
| #3-1 | SEQ ID NO.4 | SEQ ID NO. 13 | SEQ ID NO.10 | SEQ ID NO. 14 | SEQ ID NO. 12 | format 4 |
| #6-2 | SEQ ID NO.5 | SEQ ID NO. 13 | SEQ ID NO.10 | SEQ ID NO. 14 | SEQ ID NO. 12 | format 4 |
| #5-1 | SEQ ID NO.1 | SEQ ID NO. 16 | SEQ ID NO.10 | SEQ ID NO. 15 | SEQ ID NO. 12 | format 3 |

### 1.2. Expression of fusion protein

The nucleic acid sequence encoding the fusion protein was constructed into a pTT5 plasmid according to the 4 construction modes described above. Protein transient expression was performed using the ExpiCHO expression system. Host cells ExpiCHO-S (Cat No. A29127) passaged on ExpiCHOTM Expression Medium (Cat No. A2910001) were diluted to an appropriate density and placed in a shaker (100 rpm, 37 °C, 8% CO₂) to be transfected. A vector carrying the nucleic acid sequence encoding the fusion protein was added to an OptiPRO^{™} SFM (Cat No. 12309019) medium, so that the final concentration of the vector was 0.5-1.0 µg/mL. A proper amount of ExpiFectamine^{™} CHO Reagent (Cat No. A29129) was added to the OptiPRO^{™} SFM medium containing DNA to form a DNA-ExpiFectamine^{™} CHO Reagent complex, which was left to stand at room temperature for 1-5 min, and then the complex was slowly added dropwise to the cell suspension to be transfected. On day 1 after transfection, a certain amount of ExpiFectamine^{™} CHO Enhancer (Cat No. A29129) and ExpiCHO^{™} Feed (Cat No. A29129) were supplemented, and the cells were cooled to 32 °C and cultured. On days 4-6 after transfection, a certain amount of ExpiCHO^{™} Feed (Cat No. A29129) was supplemented. On days 10-12 after transfection, the cells were centrifuged at 5000 g for 30 min, and the supernatant was collected.

### 1.3. Protein purification

The variant fusion protein was purified by magnetic bead method. A proper amount of magnetic bead suspension (GenScript, Cat. NO. L00695) was added to the supernatant after fermentation, and the mixture was incubated in a rotary mixer for 2 h to ensure that the IL-15 fusion protein was bound to the magnetic bead. The supernatant was discarded, and the magnetic bead was washed for 3 times with PBS, and finally eluted with citrate (pH 3.0) to give the IL-15 fusion protein. After the sample was neutralized by 1 M Tris-Hcl, and the protein concentration was determined using NanoDrop One. IL-15 fusion proteins with a protein purity of 50 mg/mL were selected for subsequent formulation screening experiments.

### Example 2. Buffer System Screening

### 2.1. Buffer system screening protocol

The screening experiment covered 4 buffer salts (acetate, citrate, histidine, and phosphate) and 12 formulation buffer systems (pH from 4.5 to 7.5). In each formula, IL-15 fusion protein #6-1-2 (same as in Examples 3-7 below) was used, and the protein concentration was 50 mg/mL; the protein stock solution was concentrated and buffer exchanged by ultrafiltration into the corresponding 12 buffer systems. Through a high-temperature accelerated test at 40 °C, the stability of the protein in the 12 buffer systems was comprehensively investigated and compared by taking the appearance, pH value, concentration, purity (size exclusion chromatography (SE-HPLC), non-reduced capillary electrophoresis-sodium dodecyl sulfate CE-SDS (NR), and reduced capillary electrophoresis-sodium dodecyl sulfate CE-SDS (R)), charge isomerism (whole column capillary isoelectric focusing electrophoresis (iCIEF)), and dynamic light scattering (DLS) as indexes, so as to screen out an optimal buffer system. The specific formulation and investigation scheme are shown in Table 3 below.

**Table 3. Experimental protocol for formulation buffer system screening**

| No. | Buffer system, pH | | T0 | 40°C | | |
|---|---|---|---|---|---|---|
| | | | | 1W | 2W | 4W |
| F1 | 20 mM acetic acid-sodium acetate | 4.5 | X | X | X | X |
| F2 | | 5 | X | X | X | X |
| F3 | | 5.5 | X | X | X | X |
| F4 | 20 mM citric acid-sodium citrate | 5 | X | X | X | X |
| F5 | | 5.5 | X | X | X | X |
| F6 | | 6 | X | X | X | X |
| F7 | 20 mM histidine-histidine hydrochloride | 5.5 | X | X | X | X |
| F8 | | 6 | X | X | X | X |
| F9 | | 6.5 | X | X | X | X |
| F10 | 20 mM disodium hydrogen phosphate-sodium dihydrogen phosphate | 6.5 | X | X | X | X |
| F11 | | 7 | X | X | X | X |
| F12 | | 7.5 | X | X | X | X |

| | | | | | | |
|---|---|---|---|---|---|---|
| Note: for X, the following experiments were performed: iCIEF, SE-HPLC, CE-SDS(NR), CE-SDS(R), DLS T0 = investigation start point; W = week. | | | | | | |

### 2.2. Buffer system screening results

The major results of the buffer system screening are summarized in Tables 4-5 below.

**Table 4. Accelerated test results of formulation buffer system screening at 40 °C**

| NO. | Condition | DLS | SE-HPLC | | | iCIEF |
|---|---|---|---|---|---|---|
| | | Radius, nm | High molecular weight peak (%) | Main Peak (%) | Low molecular weight peak (%) | Isoelectric point |
| F1 | T0 | 4.3 | 2.2 | 97.75 | ND | 7.0-8.8 |
| | 40°C,1W | 3.2 | 3.7 | 96.16 | 0.19 | 7.0-8.8 |
| | 40°C,2W | 3.3 | 3.7 | 95.9 | 0.3 | 7.0-8.8 |
| | 40°C,4W | 3.1 | 6.2 | 93 | 0.76 | 7.0-8.8 |
| F2 | T0 | 4.6 | 2.3 | 97.66 | ND | 7.0-8.8 |
| | 40°C,1W | 4.4 | 3.1 | 96.82 | 0.1 | 7.0-8.8 |
| | 40°C,2W | 4.5 | 3.1 | 96.7 | 0.3 | 7.0-8.8 |
| | 40°C,4W | 4.1 | 5.1 | 94.44 | 0.48 | 7.0-8.8 |
| F3 | T0 | 5.8 | 2.4 | 97.57 | ND | 7.0-8.8 |
| | 40°C,1W | 5.5 | 3.4 | 96.48 | 0.13 | 7.0-8.8 |
| | 40°C,2W | 5.7 | 3.3 | 96.5 | 0.2 | 7.0-8.8 |
| | 40°C,4W | 4.9 | 5.2 | 94.41 | 0.42 | 7.0-8.8 |
| F4 | T0 | 10.5 | 2.6 | 97.34 | ND | 7.0-8.8 |
| | 40°C,1W | 9.8 | 6.5 | 93.27 | 0.23 | 7.0-8.8 |
| | 40°C,2W | 8.2 | 7.5 | 92 | 0.5 | 7.0-8.8 |
| | 40°C,4W | 8.6 | 14.7 | 84.44 | 0.86 | 7.0-8.8 |
| FS | T0 | 9.1 | 2.5 | 97.44 | ND | 7.0-8.8 |
| | 40°C,1W | 8.8 | 4.7 | 95.23 | 0.13 | 7.0-8.8 |
| | 40°C,2W | 8.6 | 5.1 | 94.6 | 0.2 | 7.0-8.8 |
| | 40°C,4W | 7.5 | 8.3 | 91.15 | 0.55 | 7.0-8.8 |
| F6 | T0 | 7.5 | 2.7 | 97.29 | ND | 7.0-8.8 |
| | 40°C,1W | 9.3 | 5.1 | 94.74 | 0.14 | 7.0-8.8 |
| | 40°C,2W | 6.7 | 5.6 | 94.2 | 0.2 | 7.0-8.8 |
| | 40°C,4W | 7.5 | 8.8 | 90.82 | 0.41 | 7.0-8.8 |
| F7 | T0 | 5.7 | 2.3 | 97.66 | ND | 7.0-8.8 |
| | 40°C,1W | 5.5 | 3.0 | 96.86 | 0.1 | 7.0-8.8 |
| | 40°C,2W | 5.5 | 3.1 | 96.7 | 0.2 | 7.0-8.8 |
| | 40°C,4W | 5.3 | 5.0 | 94.53 | 0.52 | 7.0-8.8 |
| F8 | T0 | 5.9 | 2.5 | 97.53 | ND | 7.0-8.8 |
| | 40°C,1W | 5.7 | 3.7 | 96.22 | 0.08 | 7.0-8.8 |
| | 40°C,2W | 5.7 | 3.8 | 96.1 | 0.2 | 7.0-8.8 |
| | 40°C,4W | 5.8 | 5.5 | 94.05 | 0.47 | 7.0-8.8 |
| F9 | T0 | 6.1 | 2.7 | 97.26 | ND | 7.0-8.8 |
| | 40°C,1W | 6.4 | 4.8 | 95.09 | 0.13 | 7.0-8.8 |
| | 40°C,2W | 7.3 | 4.9 | 94.9 | 0.2 | 7.0-8.8 |
| | 40°C,4W | 6.3 | 6.7 | 92.89 | 0.39 | 7.0-8.8 |
| F10 | T0 | 7.8 | 3.1 | 96.91 | ND | 7.0-8.8 |
| | 40°C,1W | 9.0 | 6.3 | 93.58 | 0.1 | 7.0-8.8 |
| | 40°C,2W | 9.1 | 7.7 | 92.1 | 0.2 | 7.0-8.8 |
| | 40°C,4W | 6.7 | 12.2 | 87.3 | 0.55 | 7.0-8.8 |
| F11 | T0 | 9.7 | 3.3 | 96.67 | ND | 7.0-8.8 |
| | 40°C,1W | 10.4 | 8.7 | 91.17 | 0.14 | 7.0-8.8 |
| | 40°C,2W | 11.4 | 11.2 | 88.5 | 0.3 | 7.0-8.8 |
| | 40°C,4W | 11.0 | 17.0 | 82.42 | 0.62 | 7.0-8.8 |
| F12 | T0 | 9.6 | 3.6 | 96.4 | ND | 7.0-8.8 |
| | 40°C,1W | 9.3 | 10.0 | 89.83 | 0.17 | 7.0-8.8 |
| | 40°C,2W | 8.8 | 13.1 | 86.6 | 0.3 | 7.0-8.8 |
| | 40°C,4W | 10.7 | 20.0 | 79.12 | 0.84 | 7.0-8.8 |

| | | | | | | |
|---|---|---|---|---|---|---|
| Note: ND = not detected. | | | | | | |

**Table 5. Accelerated test results of formulation buffer system screening at 40 °C**

| NO. | Condition | CE-SDS (NR) | | | CE-SDS (R) | |
|---|---|---|---|---|---|---|
| | | Degradation (%) | Main Peak (%) | High molecular weight (%) | Light chain + heavy chain% | Non-glycosylated heavy chain% |
| F1 | T0 | 0.57 | 99.42 | ND | 94.99 | 0.56 |
| | 40°C,1W | 1.98 | 98.02 | ND | 94.62 | 0.61 |
| | 40°C,2W | 1.9 | 96.16 | 1.93 | 94.76 | ND |
| | 40°C,4W | 10.38 | 88.35 | 1.27 | 89.61 | 0.9 |
| F2 | T0 | 0.53 | 99.47 | ND | 94.93 | 0.54 |
| | 40°C,1W | 1.72 | 98.26 | ND | 95.62 | 0.33 |
| | 40°C,2W | 1.77 | 96.08 | 2.14 | 94.73 | ND |
| | 40°C,4W | 8.98 | 89.99 | 1.03 | 91.61 | 0.96 |
| F3 | T0 | 0.58 | 99.42 | ND | 94.77 | 0.67 |
| | 40°C,1W | 1.56 | 98.45 | ND | 94.83 | 0.62 |
| | 40°C,2W | 1.75 | 95.28 | 2.97 | 94.56 | 0.46 |
| | 40°C,4W | 8.56 | 89.26 | 2.18 | 92.47 | 1.11 |
| F4 | T0 | 0.58 | 99.43 | ND | 94.38 | 0.73 |
| | 40°C,1W | 1.5 | 98.25 | 0.25 | 94.27 | 0.66 |
| | 40°C,2W | 2.24 | 94.45 | 3.31 | 93.93 | 0.44 |
| | 40°C,4W | 9.68 | 85.8 | 4.52 | 89.25 | 1.05 |
| F5 | T0 | 0.67 | 99.32 | ND | 94.88 | 0.71 |
| | 40°C,1W | 1.56 | 97.99 | 0.46 | 94.59 | 0.68 |
| | 40°C,2W | 2.43 | 93.59 | 3.99 | 94.36 | 0.39 |
| | 40°C,4W | 11.57 | 83.58 | 4.85 | 91.09 | 1.09 |
| F6 | T0 | 0.56 | 99.44 | ND | 94.72 | 0.63 |
| | 40°C,1W | 1.55 | 97.59 | 0.86 | 94.74 | 0.71 |
| | 40°C,2W | 2.4 | 94.88 | 2.71 | 94.37 | 0.46 |
| | 40°C,4W | 11.04 | 82.64 | 6.32 | 91.04 | 1.25 |
| F7 | T0 | 0.48 | 99.51 | ND | 94.79 | 0.63 |
| | 40°C,1W | 1.56 | 98.42 | ND | 94.92 | 0.65 |
| | 40°C,2W | 2.92 | 95.85 | 1.23 | 94.01 | 0.34 |
| | 40°C,4W | 10.71 | 84.42 | 4.87 | 91.55 | 1 |
| F8 | T0 | 0.48 | 99.52 | ND | 94.50 | 0.81 |
| | 40°C,1W | 1.63 | 97.93 | 0.46 | 94.83 | 0.71 |
| | 40°C,2W | 1.74 | 95.93 | 2.34 | 94.19 | 0.47 |
| | 40°C,4W | 11.71 | 82.81 | 5.48 | 92.04 | 1.02 |
| F9 | T0 | 0.40 | 99.60 | ND | 94.38 | 1.17 |
| | 40°C,1W | 1.96 | 97.01 | 1.03 | 95.06 | 0.69 |
| | 40°C,2W | 2.7 | 94.25 | 3.05 | 93.97 | 0.43 |
| | 40°C,4W | 11.14 | 81.64 | 7.22 | 90.83 | 1.22 |
| F10 | T0 | 0.41 | 99.58 | ND | 94.70 | 0.71 |
| | 40°C,1W | 1.83 | 96.63 | 1.54 | 95.06 | 0.87 |
| | 40°C,2W | 3.42 | 92.39 | 4.19 | 92.56 | 0.53 |
| | 40°C,4W | 12.7 | 77.87 | 9.43 | 78.16 | 1.78 |
| F11 | T0 | 0.50 | 99.51 | ND | 94.99 | 0.60 |
| | 40°C,1W | 2.28 | 94.69 | 3.03 | 94.93 | 0.71 |
| | 40°C,2W | 3.44 | 90.18 | 6.37 | 89.26 | 0.6 |
| | 40°C,4W | 12.56 | 74.89 | 12.55 | 75.18 | 1.99 |
| F12 | T0 | 0.42 | 99.58 | ND | 94.83 | 0.53 |
| | 40°C,1W | 2.79 | 93.12 | 4.08 | 93.46 | 1.03 |
| | 40°C,2W | 4.25 | 87.83 | 7.94 | 88.55 | 0.54 |
| | 40°C,4W | 13.21 | 74.53 | 12.26 | 70.43 | 2.16 |

| | | | | | | |
|---|---|---|---|---|---|---|
| Note: ND = not detected. | | | | | | |

### 2.3. Buffer system screening conclusion

The screening experiment for the buffer system covered 4 buffer salts (acetate, citrate, histidine, and phosphate) and 12 formulation buffer systems (pH from 4.5 to 7.5). A high-temperature accelerated test at 40 °C was performed, and the stability of the protein in the 12 buffer systems was comprehensively investigated and compared.

The study used purity (SE-HPLC, CE-SDS (NR), CE-SDS (R), charge isomerism (iCIEF), and DLS as indexes. SE-HPLC: investigation was performed at 40 °C for 4 weeks, the ranking of the SE-HPLC main peak purity and the polymer: F2, F3, F7, and F8 > F1 and F9 > F5 and F6 > F10 > F4, F11, and F12;
CE-SDS (NR): investigation was performed at 40 °C for 4 weeks, CE(NR) main peak purity F1 F2, and F3 > F4, F5, and F7 > F6 and F8 > F9 > F10, F11, and F12; degradation peak ranking: F2 and F3 > F1 and F4 > F5, F6, F7, F8, and F9 > F10, F11, and F12;
CE-SDS (R): investigation was performed at 40 °C for 4 weeks, the light chain and heavy chain content ranking: F3 and F8 > F2, F5, F6, F7, and F9 > F1 and F4 > F10, F11, and F12;
DLS: investigation was performed at 40 °C for 4 weeks, the particle size ranking: F1, F2, and F3 > F7 and F8 > F9 > F4, F5, and F6 > F10, F11, and F12.

In summary, through the buffer system screening, the ranking of the formulas was as follows: F2 and F3 > F1 and F8 > F7 > F5, F6, and F9 > F4, F10, F11, and F12; the F1, F2, F3, and F8 formulas were comprehensively superior to the other formulas in terms of purity, degradation, aggregation, charge heteroplasmon, and the like in the accelerated test at 40 °C, and showed good stability (wherein F2 and F3 were slightly superior to F1 and F8). However, since when investigation was performed at 40 °C for 4 weeks, the CE-SDS (NR) purity of all the formulas decreased dramatically (> 10%), and other items varied to different extents, the development of freeze-dried formulations were performed. Since acetic acid in the acetic acid buffer systems (F1-F3) was volatile and could not be freeze-dried, F8 (20 mM histidine-histidine hydrochloride, pH 6.0) was selected for the next development.

### Example 3. Excipient Screening

### 3. 1. Excipient screening protocol

A corresponding excipient was added to the optimal buffer system with reference to the screening results of the buffer system and the aim of developing a freeze-dried formulation, including 4 formulas in total, i.e., glycine, sucrose, trehalose, and mannitol, and the protein concentration was 50 mg/mL. The sample was freeze-dried using a vacuum freeze dryer, wherein the freeze-drying steps were as follows: (1) pre-freezing: the drying box of the vacuum freeze dryer was cooled to -45 °C in a maximum cooling method and kept for 3-5 h; (2) primary drying: the degree of vacuum in primary drying was 0.1 mBar, the temperature was kept at -30 °C to 0 °C, and the total time of the step should be greater than 30 h; (3) desorption drying: after primary drying, the temperature was raised to 25 °C at a heating rate of 0.1-0.3 °C/min, desorption drying was started, the degree of vacuum was kept at 0.1 mBar for 12-14 h, then ultimate vacuum was created, and the temperature was kept unchanged for longer than 1 h.

The freeze-dried sample was subjected to high-temperature accelerated test at 40 °C, and the stability of the formulas was investigated using purity (SE-HPLC, CE-SDS (NR), and CE-SDS (R)), charge isomerism (iCIEF), and DLS as indexes to select 1 optimal formulation formula for the next round of surfactant screening. The specific formulation and investigation scheme are shown in Table 6 below.

**Table 6. Excipient screening protocol**

| Formula | Formulation | | Type | T0 | 40°C | |
|---|---|---|---|---|---|---|
| | | | | | 2W | 4W |
| F8-1 | 20 mM histidine-histidine hydrochloride, pH 6.0 | 2% glycine, 0.04% polysorbate 80 | Freeze-dried powder | X | X | X |
| F8-2 | | 4.5% mannitol, 0.04% polysorbate 80 | | X | X | X |
| F8-3 | | 8% sucrose, 0.04% polysorbate 80 | | X | X | X |
| F8-4 | | 8% trehalose, 0.04% polysorbate 80 | | X | X | X |

| | | | | | | |
|---|---|---|---|---|---|---|
| Note: for X, the following experiments were performed: iCIEF, SE-HPLC, CE-SDS (NR), CE-SDS (R), DLS, insoluble particle, and moisture (T0) T0 = investigation start point; W = week | | | | | | |

### 3.2. Excipient screening results

The major results of the excipient screening are summarized in Tables 7-8 below.

**Table 7. Accelerated test results of excipient screening at 40°C**

| NO. | Condition | SE-HPLC | | | CE-SDS (NR) | | | CE-SDS (R) | |
|---|---|---|---|---|---|---|---|---|---|
| | | High molecular weight peak (%) | Main Peak (%) | Low molecular weight peak (%) | Degradation (%) | Main Peak (%) | High molecular weight (%) | Light chain + heavy chain% | Other% |
| F8-1 (2% glycine, 0.04% polysorbate 80) | T0 | 4.60 | 95.40 | ND | 2.11 | 97.89 | ND | 91.05 | 8.96 |
| | 40°C,2W | 8.40 | 91.60 | ND | 1.64 | 98.37 | ND | 90.87 | 9.14 |
| | 40°C,4W | 10.60 | 86.90 | 2.50 | 0.82 | 95.97 | 3.22 | 91.11 | 8.90 |
| F8-2 (4.5% mannitol, 0.04% | T0 | 5.30 | 94.70 | ND | 0.70 | 99.31 | ND | 90.73 | 9.28 |
| | 40°C,2W | 8.30 | 91.70 | ND | 1.46 | 98.53 | ND | 89.38 | 10.63 |
| polysorbate 80) | 40°C,4W | 9.00 | 89.00 | 2.00 | 0.80 | 96.38 | 2.83 | 90.88 | 9.12 |
| F8-3 (8% sucrose, 0.04% polysorbate 80) | T0 | 3.10 | 96.90 | ND | 1.08 | 98.93 | ND | 91.15 | 8.86 |
| | 40°C,2W | 4.00 | 96.00 | ND | 1.50 | 98.49 | ND | 90.14 | 9.87 |
| | 40°C,4W | 3.90 | 94.00 | 2.20 | 2.24 | 96.45 | 1.32 | 91.29 | 8.70 |
| F8-4 (8% trehalose, 0.04% polysorbate 80) | T0 | 3.00 | 97.00 | ND | 0.89 | 99.11 | ND | 90.96 | 9.03 |
| | 40°C,2W | 4.30 | 95.70 | ND | 1.72 | 98.27 | ND | 90.18 | 9.81 |
| | 40°C,4W | 4.20 | 93.20 | 2.60 | 1.63 | 97.19 | 1.17 | 90.97 | 9.02 |

| | | | | | | | | | |
|---|---|---|---|---|---|---|---|---|---|
| Note: ND = not detected. | | | | | | | | | |

**Table 8. Accelerated test results of excipient screening at 40 °C**

| NO. | Condition | DLS | | iCIEF | Insoluble particle (particles/mL) | | Moisture (%) |
|---|---|---|---|---|---|---|---|
| | | Diameter (nm) | Polydispersity index | Isoelectric point | ≥10µm | ≥25µm | |
| F8-1 (2% glycine, 0.04% polysorbate 80) | T0 | 12.39 | 0.21 | 7.256∼9.306 | 818.70 | 4.87 | 0.80 |
| | 40°C,2W | 14.55 | 0.22 | 7.269∼9.233 | 40.61 | 0.00 | NA |
| | 40°C,4W | 16.44 | 0.22 | 7.242∼9.285 | 30.86 | 0.00 | NA |
| F8-2 (4.5% mannitol, 0.04% polysorbate 80) | T0 | 10.68 | 0.24 | 7.191∼9.225 | 769.96 | 1.62 | 0.88 |
| | 40°C,2W | 11.24 | 0.24 | 7.200∼9.225 | 24.37 | 4.87 | NA |
| | 40°C,4W | 12.56 | 0.21 | 7.174∼9.278 | 1728.36 | 92.59 | NA |
| F8-3 (8% sucrose, 0.04% polysorbate 80) | T0 | 10.75 | 0.18 | 7.19∼9.221 | 583.16 | 19.49 | 0.66 |
| | 40°C,2W | 10.65 | 0.18 | 7.195∼9.228 | 25.99 | 4.87 | NA |
| | 40°C,4W | 10.87 | 0.19 | 7.170∼9.282 | 71.47 | 11.37 | NA |
| F8-4 (8% trehalose, 0.04% polysorbate 80) | T0 | 9.94 | 0.16 | 7.2∼9.221 | 186.94 | 1.62 | 0.58 |
| | 40°C,2W | 10.45 | 0.20 | 7.208∼9.221 | 34.11 | 4.87 | NA |
| | 40°C,4W | 10.95 | 0.19 | 7.180∼9.294 | 55.23 | 4.87 | NA |

| | | | | | | | |
|---|---|---|---|---|---|---|---|
| Note: NA = not applicable. | | | | | | | |

### 3.3. Excipient screening conclusion

The sample concentration was 50 mg/mL, and the excipient screening was performed in the form of a freeze-dried powder at 40 °C for 4 weeks. Investigation was performed at 40 °C for 4 weeks using purity (SE-HPLC, CE-SDS (NR), and CE-SDS (R)), charge isomerism (iCIEF), and DLS as indexes. In terms of insoluble particle, F8-2 formula was significantly higher than that of other formulas; in terms of SE-HPLC purity, main peaks of F8-1 and F8-2 significantly reduced, and the low molecular weight peaks significantly increased; in terms of DLS detection, the diameters of F8-1 and F8-2 significantly increased; for CE-SDS (NR) detection main peaks, F8-3 and F8-4 > F8-1 and F8-2; compared comprehensively, F8-3 and F8-4 formulas were comprehensively superior to the other formulas in terms of purity, degradation, aggregation, charge heteroplasmon, and the like in the accelerated test at 40 °C, and showed good stability. Therefore, formulas F8-3 and F8-4 (containing 8% sucrose (W/V) and 8% trehalose (W/V), respectively) were preliminarily selected for the next surfactant screening study.

### Example 4. 1st Round of Surfactant Screening

### 4.1. Surfactant screening protocol

Different concentrations of surfactants were added to the selected buffer system and excipients, including 0.02%, 0.04%, 0.06%, and 0.08% polysorbate 80, 8 formulas in total. The protein concentration was 50 mg/mL. By accelerated test at 40 °C, the stability of the formulas was investigated using purity (SE-HPLC), CE-SDS (NR), CE-SDS (R), charge isomerism (iCIEF), DLS, and insoluble particle as indexes to select the optimal formulation formula for formula stability verification experiment. The final formula was verified. The specific formulation and investigation scheme are shown in Table 9 below.

**Table 9. 1st round of surfactant screening protocol**

| Formula | Formulation | | T0 | 40°C | | |
|---|---|---|---|---|---|---|
| | | | | 1W | 2W | 4W |
| F8-3-1 | 20mM Histidine-histidine hydrochloride, 8% sucrose, pH 6.0 | 0.02% polysorbate 80 | X | X | X | X |
| F8-3-2 | | 0.04% polysorbate 80 | X | X | X | X |
| F8-3-3 | | 0.06% polysorbate 80 | X | X | X | X |
| F8-3-4 | | 0.08% polysorbate 80 | X | X | X | X |
| F8-4-5 | 20mM Histidine-histidine hydrochloride, 8% trehalose, pH 6.0 | 0.02% polysorbate 80 | X | X | X | X |
| F8-4-6 | | 0.04% polysorbate 80 | X | X | X | X |
| F8-4-7 | | 0.06% polysorbate 80 | X | X | X | X |
| F8-4-8 | | 0.08% polysorbate 80 | X | X | X | X |

| | | | | | | |
|---|---|---|---|---|---|---|
| Note: for X, the following experiments were performed: iCIEF, SE-HPLC, CE-SDS (NR), CE-SDS (R), DLS, and insoluble particle (T0 and investigation end point) T0 = investigation start point; W = week | | | | | | |

### 4.2. Surfactant screening results

The major results of the surfactant screening are summarized in Tables 10-11 below.

**Table 10. Accelerated test results of 1st round of surfactant screening at 40 °C**

| NO. | Condition | DLS | | SE-HPLC | | | CE-SDS (NR) | | |
|---|---|---|---|---|---|---|---|---|---|
| | | Diameter (nm) | Polydispersity index | High molecular weight peak (%) | Main Peak (%) | Low molecular weight peak (%) | Degradation (%) | Main Peak (%) | High molecular weight (%) |
| F8-3-1 | T0 | 24.32 | 0.26 | 0.8 | 99.2 | ND | 1.93 | 98.07 | ND |
| | 40°C,1W | 18.86 | 0.26 | 1.7 | 98.2 | 0.2 | 3.04 | 96.96 | ND |
| | 40°C,2W | 20.30 | 0.28 | 2.7 | 96.9 | 0.3 | 4.92 | 95.07 | ND |
| | 40°C,4W | 20.95 | 0.29 | 3.2 | 96.3 | 0.5 | 4.59 | 95.4 | ND |
| F8-3-2 | T0 | 22.84 | 0.26 | 1.3 | 98.7 | ND | 1.77 | 98.22 | ND |
| | 40°C,1W | 19.84 | 0.26 | 1.8 | 98.0 | 0.2 | 3.33 | 96.67 | ND |
| | 40°C,2W | 21.20 | 0.29 | 3.6 | 96.0 | 0.4 | 6.10 | 93.90 | ND |
| | 40°C,4W | 23.97 | 0.31 | 4.3 | 95.2 | 0.5 | 5.96 | 94.04 | ND |
| F8-3-3 | T0 | 22.17 | 0.26 | 1.4 | 98.6 | ND | 2.45 | 97.54 | ND |
| | 40°C,1W | 19.18 | 0.26 | 2.3 | 97.5 | 0.2 | 2.73 | 97.27 | ND |
| | 40°C,2W | 20.38 | 0.28 | 4.0 | 95.7 | 0.3 | 6.03 | 93.97 | ND |
| | 40°C,4W | 25.04 | 0.32 | 6.0 | 93.5 | 0.5 | 6.01 | 92.55 | 1.43 |
| F8-3-4 | T0 | 21.55 | 0.26 | 1.8 | 98.2 | ND | 2.39 | 97.61 | ND |
| | 40°C,1W | 18.33 | 0.27 | 2.8 | 97.0 | 0.2 | 3.44 | 96.55 | ND |
| | 40°C,2W | 19.88 | 0.28 | 4.3 | 95.4 | 0.3 | 6.26 | 93.74 | ND |
| | 40°C,4W | 26.80 | 0.31 | 8.0 | 91.5 | 0.5 | 8.83 | 89.49 | 1.68 |
| F8-4-5 | T0 | 24.02 | 0.27 | 0.8 | 99.2 | ND | 2.42 | 97.58 | ND |
| | 40°C,1W | 19.00 | 0.26 | 1.7 | 98.1 | 0.2 | 4.28 | 95.70 | ND |
| | 40°C,2W | 20.83 | 0.28 | 2.8 | 96.9 | 0.3 | 6.54 | 93.46 | ND |
| | 40°C,4W | 22.52 | 0.30 | 3.5 | 96.1 | 0.4 | 6.36 | 91.88 | 1.74 |
| F8-4-6 | T0 | 24.60 | 0.26 | 1.1 | 98.9 | ND | 2.53 | 97.48 | ND |
| | 40°C,1W | 20.45 | 0.26 | 2.0 | 97.8 | 0.2 | 4.37 | 95.63 | ND |
| | 40°C,2W | 21.29 | 0.29 | 3.6 | 96.0 | 0.3 | 7.28 | 92.73 | ND |
| | 40°C,4W | 23.71 | 0.31 | 4.6 | 95.0 | 0.5 | 4.79 | 93.43 | 1.79 |
| F8-4-7 | T0 | 24.31 | 0.26 | 1.0 | 99.0 | ND | 2.57 | 97.43 | ND |
| | 40°C,1W | 19.99 | 0.26 | 2.6 | 97.2 | 0.2 | 3.5 | 96.49 | ND |
| | 40°C,2W | 20.14 | 0.28 | 4.7 | 95.0 | 0.3 | 6.14 | 93.86 | ND |
| | 40°C,4W | 25.27 | 0.32 | 6.2 | 93.4 | 0.5 | 5.52 | 92.13 | 2.36 |
| F8-4-8 | T0 | 23.81 | 0.27 | 1.3 | 98.7 | ND | 2.82 | 97.2 | ND |
| | 40°C,1W | 19.66 | 0.26 | 3.8 | 96.0 | 0.2 | 4.31 | 95.69 | ND |
| | 40°C,2W | 19.70 | 0.29 | 5.7 | 93.9 | 0.3 | 6.73 | 93.26 | ND |
| | 40°C,4W | 24.26 | 0.32 | 7.7 | 91.9 | 0.5 | 5.05 | 92.83 | 2.13 |

| | | | | | | | | | |
|---|---|---|---|---|---|---|---|---|---|
| Note: ND = not detected. | | | | | | | | | |

**Table 11. Accelerated test results of 1st round of surfactant screening at 40 °C**

| NO. | Condition | CE-SDS (R) | | iCIEF | Insoluble particle (particles/mL) | |
|---|---|---|---|---|---|---|
| | | Light chain + heavy chain% | Other% | Isoelectric point | ≥10µm | ≥25µm |
| F8-3-1 | T0 | 97.35 | 2.65 | 7.219∼8.477 | 2.27 | 0.00 |
| | 40°C,1W | 96.81 | 3.20 | 7.183∼8.450 | NA | NA |
| | 40°C,2W | 96.51 | 3.49 | 7.182∼8.459 | NA | NA |
| | 40°C,4W | 94.73 | 5.26 | 7.095∼8.458 | 15.88 | 0.00 |
| F8-3-2 | T0 | 97.72 | 2.27 | 7.216∼8.454 | 11.35 | 0.00 |
| | 40°C,1W | 96.74 | 3.25 | 7.181∼8.440 | NA | NA |
| | 40°C,2W | 93.82 | 6.17 | 7.195∼8.490 | NA | NA |
| | 40°C,4W | 94.11 | 5.91 | 7.102∼8.458 | 6.81 | 4.54 |
| F8-3-3 | T0 | 97.78 | 2.23 | 7.221∼8.488 | 120.26 | 22.69 |
| | 40°C,1W | 96.81 | 3.21 | 7.188∼8.471 | NA | NA |
| | 40°C,2W | 94.36 | 5.64 | 7.171∼8.453 | NA | NA |
| | 40°C,4W | 92.62 | 7.38 | 7.071∼8.465 | 4.54 | 0.00 |
| F8-3-4 | T0 | 97.44 | 2.56 | 7.220∼8.494 | 9.08 | 2.27 |
| | 40°C,1W | 96.84 | 3.15 | 7.180∼8.467 | NA | NA |
| | 40°C,2W | 94.06 | 5.93 | 7.203∼8.463 | NA | NA |
| | 40°C,4W | 92.34 | 7.68 | 7.064∼8.441 | 15.88 | 2.27 |
| F8-4-5 | T0 | 97.58 | 2.42 | 7.219∼8.485 | 4.54 | 0.00 |
| | 40°C,1W | 96.92 | 3.07 | 7.174∼8.468 | NA | NA |
| | 40°C,2W | 93.96 | 6.03 | 7.205∼8.457 | NA | NA |
| | 40°C,4W | 95.41 | 4.59 | 7.094∼8.451 | 11.35 | 0.00 |
| F8-4-6 | T0 | 97.77 | 2.24 | 7.220∼8.540 | 31.77 | 0.00 |
| | 40°C,1W | 97.44 | 2.56 | 7.185∼8.431 | NA | NA |
| | 40°C,2W | 93.02 | 6.97 | 7.206∼8.493 | NA | NA |
| | 40°C,4W | 94.07 | 5.92 | 7.092∼8.455 | 31.77 | 2.27 |
| F8-4-7 | T0 | 97.23 | 2.77 | 7.216∼8.499 | 9.08 | 0.00 |
| | 40°C,1W | 97.23 | 2.77 | 7.191∼8.482 | NA | NA |
| | 40°C,2W | 92.42 | 7.59 | 7.204∼8.476 | NA | NA |
| | 40°C,4W | 92.89 | 7.08 | 7.084∼8.448 | 4.54 | 2.27 |
| F8-4-8 | T0 | 97.51 | 2.49 | 7.196∼8.456 | 2.27 | 2.27 |
| | 40°C,1W | 95.90 | 4.10 | 7.184∼8.471 | NA | NA |
| | 40°C,2W | 92.99 | 7.00 | 7.201∼8.465 | NA | NA |
| | 40°C,4W | 92.43 | 7.55 | 7.090∼8.459 | 11.35 | 0.00 |

| | | | | | | |
|---|---|---|---|---|---|---|
| Note: NA = not applicable. | | | | | | |

### 4.3. Surfactant screening conclusion

Investigation was performed at 40 °C for 4 weeks using purity (SE-HPLC, CE-SDS (NR), and CE-SDS (R)), charge isomerism (iCIEF), DLS, and insoluble particle as indexes. In the SE-HPLC detection, the main peak content of formulas F8-3-1 and F8-4-5 were superior than that of the other formulas; in the CE-SDS (NR) detection, formula F8-3-1 was superior to the other formulas; in the CE-SDS (R) detection, formula F8-4-5 was superior to the other formulas; there was no significant difference in other items; compared comprehensively, formulas F8-3-1 and F8-4-5 were superior to the other formulas in the accelerated test at 40 °C, and showed good stability. In this round of screening, the high molecular weight peak of SE-HPLC increased with increasing polysorbate 80 concentration. Another type of surfactant, poloxamer 188, was therefore used for 2nd round of surfactant screening.

### Example 5. 2nd Round of Surfactant Screening

According to the clinical requirement, and for avoiding instability caused by aggregation of protein molecules and the like under high concentration, the following examples were carried out by reducing the protein concentration on the formula result screened by high protein concentration on the basis of the formulation formula screening described above, and the specific formula was as follows,

The glass transition temperature of the two formulas of freeze-dried powder, 25 mg/mL protein, 20 mM histidine-histidine hydrochloride, 8% sucrose (W/V), 0.04% poloxamer 188 (W/V), pH 6.0 and 25 mg/mL protein, 20 mM histidine-histidine hydrochloride, 8% trehalose (W/V), 0.04% poloxamer 188 (W/V), pH 6.0 was detected to be 74.06 °C and 115.44 °C, respectively. The glass transition temperature of the freeze-dried powder formula containing trehalose was much higher than that of the formula containing sucrose, therefore trehalose was used in the 2nd round of surfactant screening.

### 5.1. Surfactant screening protocol

Different concentrations of surfactants were added to the selected and verified buffer system and excipients, including 0.01%, 0.02%, 0.04%, 0.06%, and 0.08% poloxamer 188 (W/V), 5 formulas in total. By accelerated test at 40 °C, the stability of the formulas was investigated using purity (SE-HPLC), CE-SDS (NR), CE-SDS (R), charge isomerism (iCIEF), DLS, and insoluble particle as indexes to select the optimal formulation formula for formula stability verification experiment. The final formula was verified. The specific formulation and investigation scheme are shown in Table 12 below.

**Table 12. 2nd round of surfactant screening protocol**

| Formula | Formulation | | T0 | 40°C | | |
|---|---|---|---|---|---|---|
| | | | | 1W | 2W | 4W |
| F8-4-S1 | 20mM Histidine-histidine hydrochloride, 8% trehalose, pH 6.0 | 0.01% poloxamer 188 | X | X | X | X |
| F8-4-S2 | | 0.02% poloxamer 188 | X | X | X | X |
| F8-4-S3 | | 0.04% poloxamer 188 | X | X | X | X |
| F8-4-S4 | | 0.06% poloxamer 188 | X | X | X | X |
| F8-4-S5 | | 0.08% poloxamer 188 | X | X | X | X |

| | | | | | | |
|---|---|---|---|---|---|---|
| Note: for X, the following experiments were performed: iCIEF, SE-HPLC, CE-SDS (NR), CE-SDS (R), DLS, and insoluble particle (T0 and investigation end point) T0 = investigation start point; W = week | | | | | | |

### 5.2. Surfactant screening results

The major results of the surfactant screening are summarized in Tables 13-14 below.

**Table 13. Accelerated test results of 2nd round of surfactant screening at 40 °C**

| NO. | Condition | DLS | | SE-HPLC | | | CE-SDS (NR) | | |
|---|---|---|---|---|---|---|---|---|---|
| | | Diameter (nm) | Polydispersity index | High molecular weight peak (%) | Main Peak (%) | Low molecular weight peak (%) | Degradation (%) | Main Peak (%) | High molecular weight (%) |
| F8-4-S1 | T0 | 16.37 | 0.25 | 1.1 | 98.9 | ND | 1.54 | 98.45 | ND |
| | 40°C,1W | 14.97 | 0.26 | 1.3 | 98.6 | 0.1 | 2.78 | 96.99 | 0.24 |
| | 40°C,2W | 15.99 | 0.26 | 1.3 | 98.4 | 0.2 | 2.73 | 97.29 | ND |
| | 40°C,4W | 16.11 | 0.25 | 2.6 | 97 | 0.4 | 3.69 | 96.3 | ND |
| F8-4-S2 | TO | 16.88 | 0.24 | 1 | 99 | ND | 1.23 | 98.77 | ND |
| | 40°C,1W | 14.73 | 0.26 | 1.3 | 98.5 | 0.1 | 3.12 | 96.88 | ND |
| | 40°C,2W | 15.81 | 0.26 | 1.3 | 98.5 | 0.2 | 2.62 | 97.4 | ND |
| | 40°C,4W | 14.55 | 0.25 | 2.4 | 97.2 | 0.4 | 3.57 | 96.43 | ND |
| F8-4-S3 | T0 | 20.32 | 0.24 | 0.9 | 99.1 | ND | 1.56 | 98.44 | ND |
| | 40°C,1W | 15.67 | 0.26 | 1.4 | 98.5 | 0.1 | 3.05 | 96.95 | ND |
| | 40°C,2W | 16.51 | 0.26 | 1.3 | 98.5 | 0.2 | 2.06 | 97.95 | ND |
| | 40°C,4W | 15.32 | 0.26 | 2.3 | 97.3 | 0.4 | 3.39 | 96.6 | ND |
| F8-4-S4 | T0 | 18.24 | 0.24 | 1 | 99 | ND | 1.45 | 98.56 | ND |
| | 40°C,1W | 16.32 | 0.25 | 1.2 | 98.7 | 0.1 | 2.09 | 97.82 | 0.08 |
| | 40°C,2W | 16.02 | 0.26 | 1.3 | 98.5 | 0.2 | 2.54 | 97.46 | ND |
| | 40°C,4W | 15.04 | 0.24 | 2.3 | 97.3 | 0.4 | 3.62 | 96.39 | ND |
| F8-4-S5 | T0 | 18.83 | 0.24 | 0.9 | 99.1 | ND | 1.82 | 98.18 | ND |
| | 40°C,1W | 16.24 | 0.25 | 1.3 | 98.6 | 0.1 | 1.59 | 98.38 | ND |
| | 40°C,2W | 14.81 | 0.25 | 1.3 | 98.4 | 0.2 | 2.78 | 97.22 | ND |
| | 40°C,4W | 15.13 | 0.25 | 2.2 | 97.4 | 0.4 | 3.76 | 96.24 | ND |

| | | | | | | | | | |
|---|---|---|---|---|---|---|---|---|---|
| Note: ND = not detected | | | | | | | | | |

**Table 14. Accelerated test results of 2nd round of surfactant screening at 40 °C**

| NO. | Condition | CE-SDS (R) | | | iCIEF | Insoluble particle (particles/mL) | |
|---|---|---|---|---|---|---|---|
| | | Light chain + heavy chain% | Non-glycosylated heavy chain% | Fragment% | Isoelectric point | ≥10µm | ≥25µm |
| F8-4-S1 | T0 | 98.63 | 0.54 | 0.66 | 7.014∼8.536 | 27.23 | 0.00 |
| | 40°C,1W | 97.91 | 0.53 | 0.71 | 7.025∼8.477 | NA | NA |
| | 40°C,2W | 97.26 | 0.93 | 1.46 | 7.012∼8.521 | NA | NA |
| | 40°C,4W | 95.84 | 1.14 | 1.65 | 7.72∼8.257 | 20.42 | 0 |
| F8-4-S2 | T0 | 98.45 | 0.58 | 0.7 | 7.023∼8.543 | 18.15 | 0.00 |
| | 40°C,1W | 97.2 | 0.47 | 0.76 | 7.027∼8.480 | NA | NA |
| | 40°C,2W | 97.87 | 0.75 | 1.11 | 7.016∼8.516 | NA | NA |
| | 40°C,4W | 96.23 | 1.07 | 1.66 | 7.722∼8.26 | 6.81 | 0 |
| F8-4-S3 | T0 | 98.78 | 0.45 | 0.42 | 7.012∼8.512 | 13.61 | 4.54 |
| | 40°C,1W | 97.77 | 0.5 | 0.69 | 7.029∼8.463 | NA | NA |
| | 40°C,2W | 97.78 | 0.98 | 1.07 | 7.012∼8.525 | NA | NA |
| | 40°C,4W | 96.21 | 1.08 | 1.75 | 7.713∼8.26 | 6.81 | 0 |
| F8-4-S4 | T0 | 98.7 | 0.51 | 0.55 | 7.017∼8.526 | 22.69 | 6.81 |
| | 40°C,1W | 97.59 | 0.54 | 0.8 | 7.016∼8.478 | NA | NA |
| | 40°C,2W | 97.99 | 0.83 | 0.99 | 7.018∼8.519 | NA | NA |
| | 40°C,4W | 95.87 | 1.04 | 1.81 | 7.72∼8.254 | 11.35 | 0 |
| F8-4-S5 | T0 | 98.47 | 0.56 | 0.62 | 7.012∼8.543 | 15.88 | 2.27 |
| | 40°C,1W | 98.39 | 0.43 | 0.73 | 7.022∼8.459 | NA | NA |
| | 40°C,2W | 97.78 | 0.88 | 1.06 | 7.022∼8.521 | NA | NA |
| | 40°C,4W | 95.41 | 1.32 | 2.33 | 7.718∼8.26 | 47.65 | 2.27 |

| | | | | | | | |
|---|---|---|---|---|---|---|---|
| Note: NA = not applicable. | | | | | | | |

### 5.3. Surfactant screening conclusion

Investigation was performed at 40 °C for 4 weeks using purity (SE-HPLC, CE-SDS (NR), and CE-SDS (R)), charge isomerism (iCIEF), DLS, and insoluble particle as indexes. In the CE-SDS (R) detection, the fragment peak of formula F8-4-S5 was slightly higher than that of the other formulas, and the ratio of light chain plus heavy chain was slightly lower; there was no significant difference in other items; compared comprehensively, formulas F8-4-S1, F8-4-S2, F8-4-S3, and F8-4-S4 were superior to the other formulas in the accelerated test at 40 °C, and showed good stability. Formula F8-4-S3 was selected as the final formula (20 mM histidine-histidine hydrochloride, 8% trehalose (W/V), 0.04% poloxamer 188 (W/V), pH 6.0) in consideration of operable space of production.

### Example 6. Method for Preparing IL-15 Variant Fusion Protein Freeze-Dried Formulation of Final Formula

The IL-15 variant fusion protein solution with the protein concentration of 25 mg/mL in the final formula was aseptically aliquoted into vials. The vials were half stoppered and freeze-dried to give an IL-15 variant fusion protein freeze-dried formulation. In this example, a vacuum freeze dryer was used to freeze-dry the IL-15 variant fusion protein solution. The freeze-drying step comprises: (1) the drying box of the vacuum freeze dryer was cooled to -45 °C and kept for 3-5 h; (2) the drying box was heated to -5 °C and kept for 3-5 h; (3) the drying box was cooled to -45 °C and kept for 3-5 h. In the pre-freezing process, the heating rate and the cooling rate were kept at 0.5-1 °C/min; (4) the degree of vacuum in primary drying was 0.1 mBar, the temperature was kept at -30 °C to -20 °C, and the total time of the step should be greater than 30 h; (5) after primary drying, the temperature was raised to 25 °C at a heating rate of 0.1-0.3 °C/min, desorption drying was started, the degree of vacuum was kept at 0.1 mBar for 1-2 h, then ultimate vacuum was created, and the temperature was kept unchanged for longer than 15 h. The steps (1) to (3) were pre-freezing; (4) was primary drying; (5) was desorption drying.

### Example 7. Stability Test

An IL-15 variant fusion protein freeze-dried formulation was prepared using the preparation method of Example 6, and subjected to accelerated stability investigation at 40 °C. Main indexes include moisture, purity after reconstitution, charge isomerism, insoluble particle, activity, and the like. Based on the results of the buffer system, excipient, and surfactant screening, 1 formula was determined: 25 mg/mL IL-15 variant fusion protein, 20 mM histidine-histidine hydrochloride, 8% trehalose (W/V), 0.04% poloxamer 188 (W/V), pH 6.0, which was aliquoted into vials and freeze-dried. A stability test at 40 °C for 14 days was performed using a closed packaging system composed of a rubber stopper and an aluminum-plastic cap.

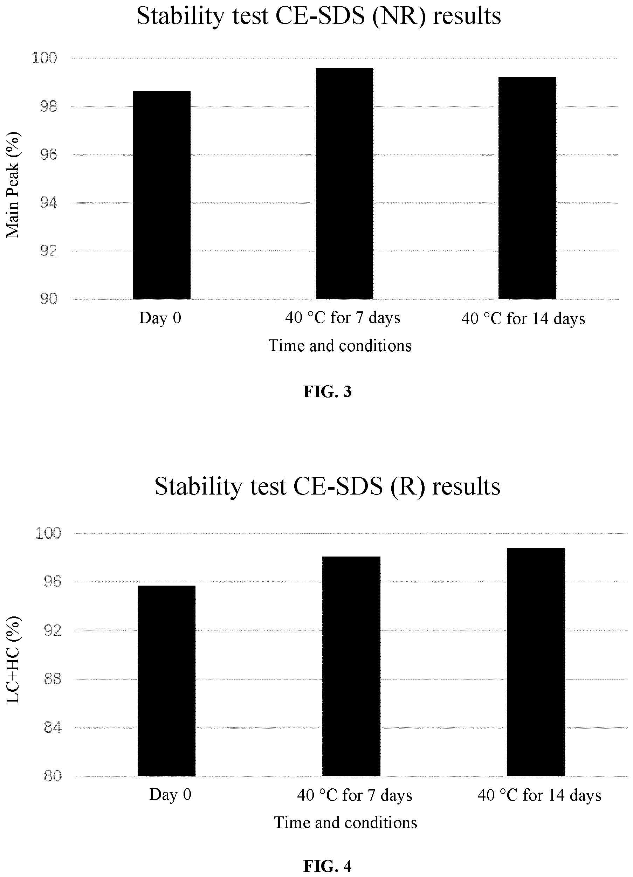

The results are shown in Tables 15-16 below and FIGs. 2-5.

**Table 15. Stability test results**

| Condition | SE-HPLC | | | CE-SDS (NR) | | | CE-SDS (R) | | |
|---|---|---|---|---|---|---|---|---|---|
| | High molecular weight peak (%) | Main Peak (%) | Low molecular weight peak (%) | Degradation (%) | Main Peak (%) | High molecular weight (%) | Light chain + heavy chain% | Non-glycosylated heavy chain% | Fragment% |
| T0 | 0.30 | 99.70 | ND | 1.36 | 98.64 | ND | 95.69 | 0.6 | 1.44 |
| 40°C,7 | 0.37 | 99.63 | ND | 0.43 | 99.57 | ND | 98.06 | 0.49 | 0.61 |
| 40°C,14 | 0.64 | 99.36 | ND | 0.77 | 99.23 | ND | 98.78 | 0.51 | 0.26 |

| | | | | | | | | | |
|---|---|---|---|---|---|---|---|---|---|
| Note: T0 = investigation start point; ND = not detected. | | | | | | | | | |

**Table 16. Stability test results**

| Condition | iCIEF | | | Insoluble particle (particles/mL) | | Activity | Moisture (%) |
|---|---|---|---|---|---|---|---|
| | Acidic peak area% | Main peak area% | Basic peak area% | ≥10µm | ≥25µm | Enzyme-linked immunosorbent assay% | |
| T0 | 30.3 | 69.7 | 0.1 | 24.37 | 0.00 | 119.32 | 0.75 |
| 40°C,7 | 29.1 | 70.5 | 0.5 | 16.24 | 1.62 | 99.59 | 0.86 |
| 40°C,14 | 27.3 | 72.2 | 0.6 | 13.00 | 1.62 | 105.63 | 1.03 |

| | | | | | | | |
|---|---|---|---|---|---|---|---|
| Note: T0 = investigation start point | | | | | | | |

Accelerated stability test at 40 °C was performed for 14 days. Purity after reconstitution (SE-HPLC) was slightly reduced, and there was no significant change in CE-SDS (NR), CE-SDS (R), charge isomerism, insoluble particle, and activity. This indicates that the protein in the formula had relatively good stability, and the aim of the present invention was achieved.

### Example 8. Effect of Test Drug on T Cell Proliferation and IFN-γ Secretion Determined by Mixed Lymphocyte Reaction (MLR)

In Examples 8-11, "#6-1" corresponds to #6-1-2 in "TERMINOLOGY AND DEFINITIONS" and "Table 1"; "aPDL1" corresponds to PD-L1-2-2; "aHeL-IL15" is an isotype control fusion protein, with the structure identical to #6-1, except that the antibody variable region is partially replaced with the variable region of an anti-chicken egg white lysozyme antibody, and the remaining sequence is identical.

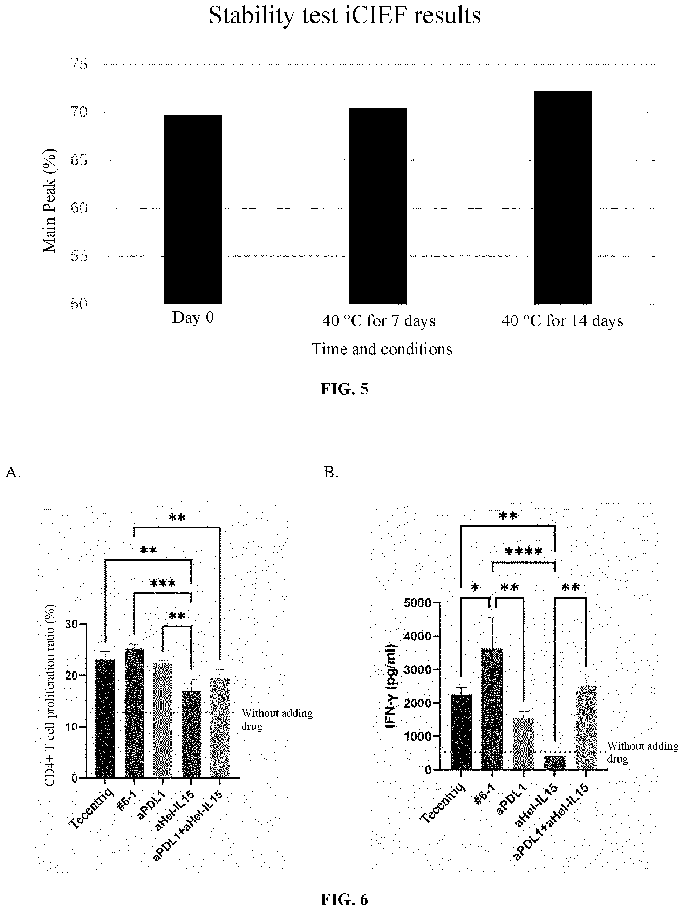

The ability of the fusion protein to enhance T cell activity was determined by mixed lymphocyte reaction (MLR). CD14⁺ monocytes were isolated from peripheral blood mononuclear cells (PBMC) of healthy human donor 1, and induced *in vitro* to differentiate into dendritic cells (DCs) using recombinant human granulocyte-macrophage colony stimulating factor (GM-CSF, Peprotech, Cat. 300-03) and recombinant human interleukin 4 (rhIL-4, Peprotech, Cat. 200-04). Mature DCs were stimulated by the addition of LPS (Sigma, Cat: L4516) on day 6 of culture. On day 7, DC cells of donor 1 were mixed and co-cultured with fluorescently labeled (Celltrace Violet Cell Proliferation Kit, Invitrogen, Cat. C34557) CD4⁺ T cells enriched from PBMCs of healthy donor 2 at a DC:CD4⁺ T cell ratio of 1: 10, the test drug at a final concentration of 2.8 nM was added, and the cells were cultured for 4 days. After 4 days, the cells were collected, the proliferation ratio of CD4⁺ T cells was detected using a flow cytometer, meanwhile, the cell culture supernatant was collected, and the content of IFN-γ in the supernatant was detected using ELISA method. As shown in FIG. 6 (one-way ANOVA, *P<0.05, **P<0.01, ***P<0.005, ****p<0.0005), compared with each control group, the #6-1 fusion protein group had higher proliferation of CD4⁺ T cells and secretion of IFN-γ. Compared with the isotype control molecule aHel-IL15, #6-1 could significantly enhance the proliferation of CD4⁺ T cells in the MLR experiment and significantly enhance the secretion of IFN-γ. The results indicate that the #6-1 fusion protein could enhance the function of T cells in mixed lymphocyte reaction.

### Example 9. Effect of Test Drug on Human Myeloid-Derived Suppressor Cell Proliferation

Cryopreserved commercial human PBMCs (Xuanfeng, Cat. No. XFB-HP050B) were resuscitated, centrifuged, resuspended with RPMI 1640 complete medium (formula: 89% RPMI 1640 medium (Gibco, Cat. No. 72400047) + 10% fetal bovine serum FBS (Gibco, Cat. No. 10099-141C) + 1% penicillin-streptomycin diabody (Gibco, Cat. No. 15070063)) to give a cell suspension, and counted. The cell density was adjusted to 6 × 10⁶/mL, the cell suspension was plated in a 96-well U-bottom plate, with 100 µL per well. Then 100 µL of the test drugs #6-1, #9-1 (see Table 1 for structural sequence), and aPDL1 2× serially diluted were added to each well. The final concentration of the fusion protein in the system was independently 500000 pM, 100000 pM, 20000 pM, 4000 pM, 800 pM, 160 pM, 32 pM, 6.4 pM, 1.28 pM, and 0.32 pM. A negative control well No treatment was also set, i.e., 100 µL of the complete medium was supplemented. The total volume of the system was 200 µL/well, and replicate wells were set. The cells and the test fusion protein were mixed well by gently pipetting using a multi-channel pipette. After they were mixed well, the well plate was placed in a 5% CO₂ incubator and cultured at 37 °C for 3 days. After the culture was completed, the well plate was taken out. The cells were pipetted using a multi-channel pipette and transferred to a new 96-well V-bottom plate. The well plate was washed once using a DPBS solution, and the solution for washing the plate was also transferred to the corresponding 96 wells. Then 100 µL/well Accutase solution (Sigma, Cat. No. A6964) was added, and the plate was placed in an incubator at 37 °C to digest the adherent cells for 10 min. The digested adherent cells were pipetted using a multi-channel pipette and transferred to the 96-well V-bottom plate described above. The plate was centrifuged at 350×g for 4 min, and the supernatant was discarded. 100 µL of diluted LIVE/DEAD Aqua dye (Invitrogen, Cat. No. L34966, 1:500 diluted with DPBS) was added to each well, and the cells were stained at 4 °C for 20 min. The plate was washed twice with a staining buffer (DPBS solution containing 2% FBS). The plate was centrifuged at 350 g for 4 min, and the supernatant was discarded. 50 µL/well of diluted Human TruStain FcX (Biolegend, Cat. No. 422302, 2 µL/test) was added, and the cells were stained at room temperature for 10 min. Then 50 µL/well of a prepared surface antibody mixture was directly added. The surface antibodies include Alexa Fluor^{®} 700 anti-human CD3 Antibody (Biolegend, Cat. No. 300424, 1µL/test), PerCP-Cy^{™}5.5 Mouse Anti-Human HLA-DR (BD, Cat. No. 560652, 1µL/test), PE-Cyanine7 anti-human CD33 (eBioscience, Cat. No. 25-0338-42, 2µL/test), BV421 Mouse Anti-Human CD11b (BD, Cat. No. 562632, 1µL/test), and FITC anti-human CD14 Antibody (Biolegend, Cat. No. 301804, 1µL/test). The cells were stained at 4 °C for 30 min. The plate was washed twice with the staining buffer. The plate was centrifuged at 350 g for 4 min, and the supernatant was discarded. 100 µL of a diluted fixative solution in a Foxp3/transcription factor staining buffer kit (eBioscience, Cat. No. 00-5523-00) was added to each well, and the mixture was fixed at 4 °C for 60 min. The plate was washed twice with 1*Permeabilization solution in the kit. The centrifugation speed was increased to 400 g for 4 min, and the supernatant was discarded. Then 100 µL/well of a prepared intracellular antibody APC anti-human Ki-67 Antibody (Biolegend, Cat. No. 350514, 1 µL/test) was slowly added, and the mixture was reacted at 4 °C for 40 min. APC-Mouse IgG1 kappa isotype control antibody (eBioscience, Cat. No. 17-4714-82) at an equivalent mass concentration was prepared. The cells were washed twice with 1*Permeabilization solution. 150 µL of the staining buffer was added to each well to resuspend the cells. Signals were detected by a flow cytometer (Thermo Attune NxT).

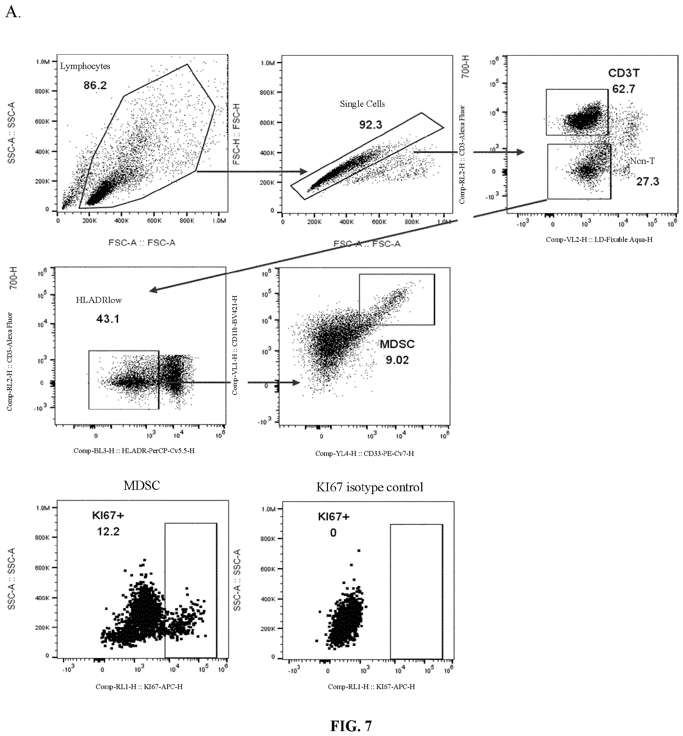

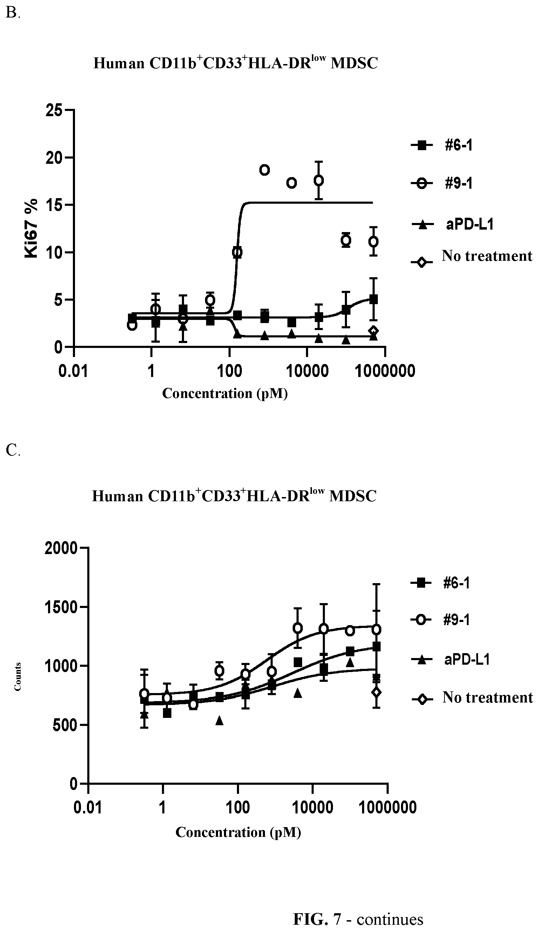

FIG. A in FIG. 7 shows gating strategy to analyze proliferation of human myeloid-derived suppressor cells (MDSCs). The Ki-67 ratio in human MDSCs (HLADR^{low} CD33⁺CD11b⁺) was analyzed. The sample treated with the highest concentration of #9-1 is shown in the figure, and a relatively significant Ki-67 positive cell population can be seen in MDSCs. FIG. B and FIG. C show the effect curve of three tested drugs on MDSC cell proliferation. FIG. B shows the percentage of Ki-67. FIG. C shows the absolute cell count of MDSCs. The results show that #9-1 could promote the proliferation of human CD33⁺ MDSCs, whereas #6-1 had almost no effect. The #6-1 molecule did not activate immunosuppressive cells.

### Example 10. Evaluation of Efficacy Advantage of Test Drug in Mice

5 × 10⁶ cells/100 µL of human melanoma cells A375 cells (purchased from Beina Bio, Cat. No. BNCC100266) were subcutaneously inoculated into the right back of NPG mice (purchased from Beijing Vitalstar Biotechnology Co., Ltd.). The day after inoculation of A375, cryopreserved PBMCs (purchased from Shanghai Rubai Biotechnology Co., Ltd., Cat. No. PBMNC100C) were resuscitated and injected into the NPG mice via the tail vein at 5 × 10⁶ cells/200 µL. 6 days after PBMC inoculation, 40 µL of blood was collected to measure the hCD45⁺ cell ratio. After the tumor grew to approximately 63 mm³, mice with too big and too small body weight, hCD45⁺ cell ratio, and tumor volume were removed, and the mice were randomized into 5 groups of PBS group, Tecentriq group (10 mg/kg), Tecentriq + aHel-IL15 group (3.4 mg/kg + 4.5 mg/kg), aHel-IL15 group (3 mg/kg), and #6-1 group (3 mg/kg) according to tumor volume. There were a total of 40 mice, with 8 mice in each group. The administration mode was intraperitoneal administration. According to the grouping, for Tecentriq + aHel-IL15 group (3.4 mg/kg + 4.5 mg/kg), aHel-IL15 group (3 mg/kg), and #6-1 group (3 mg/kg), administration was carried out on the day of grouping for a total of 1 dose; for PBS group and Tecentriq group (10 mg/kg), administration was carried out twice a week for a total of 4 doses. The tumor volume was measured 3 times a week, and data were recorded. The tumor volume (long diameter × short diameter²/2) and tumor growth inhibition rate (TGI_{TV} (%) = [1 - (mean tumor volume of treatment group on day i of administration - mean tumor volume of treatment group on day 0 of administration) / (mean tumor volume of solvent control group on day i of administration - mean tumor volume of solvent control group on day 0 of administration)] × 100%) were calculated. On day 15 of grouping administration, the administration groups all showed a significant inhibition effect on tumor volume with statistical difference (P < 0.05).

**Table 17. Effect of test drug on A375 tumor volume in immune-reconstituted NPG mice**

| **Group** | **Test drug** | **Tumor volume (mm³)^{a}** | | | **P^{b}** | **P^{c}** | **P^{d}** |
|---|---|---|---|---|---|---|---|
| | | **Day 0** | **Day 15** | **TGI_{TV} (%)** | | | |
| G1 | PBS | 63±4 | 834±76 | - | - | <0.0001 | <0.0001 |
| G2 | Tecentriq | 63±3 | 621±116 | 27.6 | <0.0001 | - | 0.9999 |
| G3 | Tecentriq+ aHel-IL15 | 64±3 | 272±52 | 73.0 | <0.0001 | 0.0001 | 0.0002 |
| G4 | aHel-IL15 | 64±4 | 571±84 | 34.1 | <0.0001 | 0.9999 | - |
| G5 | #6-1 | 64±4 | 358±85 | 61.9 | <0.0001 | 0.0051 | 0.0068 |

| | | | | | | | |
|---|---|---|---|---|---|---|---|
| Note: a: mean ± standard error; b: the statistical comparison of tumor volumes of the administration group with those in the PBS control group on day 15 of the grouping administration, Two-way ANOVA analysis, *P < 0.05, **P < 0.01, ***P < 0.001, ****P < 0.0001. 1; c: the statistical comparison of tumor volumes of the administration group with those in the positive drug Tecentriq group on day 15 of the grouping administration, Two-way ANOVA analysis. d: the statistical comparison of tumor volumes of the administration group with those in the positive drug aHel-IL15 group on day 15 of the grouping administration, Two-way ANOVA analysis. | | | | | | | |

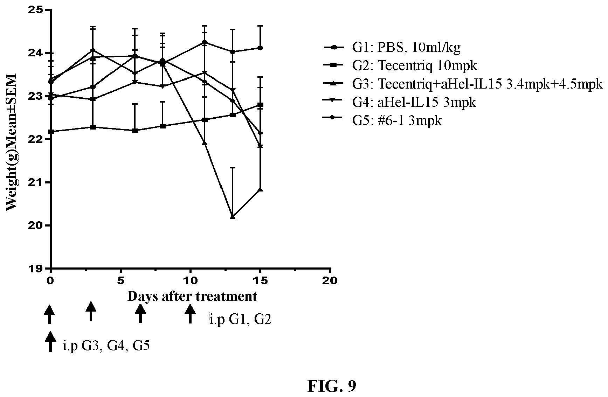

The results of body weight monitoring and observation show that the experimental animals in the aHel-IL15 (3 mg/kg) group and the #6-1 (3 mg/kg) group had good activity and feeding status during administration, and there was a downward trend in the body weight; animal death occurred in the Tecentriq + aHel-IL15 group (3.4 mg/kg + 4.5 mg/kg) on day 15 day of administration, and the death of mouse may be related to lymphokine storm caused by IL15, see FIG. 9 and Table 18.

**Table 18. Effect of test drug on body weight of immune-reconstituted A375 tumor-bearing NPG mice**

| **Group** | **Test drug** | **Body weight (g)^{a}** | | | **Body weight change (%)** | **Death statistics** |
|---|---|---|---|---|---|---|
| | | **Day 0** | **Day 15** | **P^{b}** | | |
| G1 | PBS | 23.0±0.5 | 24.1±0.5 | - | 1.1 | 0 |
| G2 | Tecentriq | 22.2±0.6 | 22.8±0.6 | 0.0018 | 0.6 | 0 |
| G3 | Tecentriq+ aHel-IL15 | 23.4±0.6 | 20.8±1.0 | 0.0102 | -2.6 | 1 |
| G4 | aHel-IL15 | 23.0±0.6 | 21.8±0.9 | 0.1625 | -1.2 | 0 |
| G5 | #6-1 | 23.3±0.5 | 22.1±1.1 | 0.5937 | -1.2 | 0 |

| | | | | | | |
|---|---|---|---|---|---|---|
| Note: a: mean ± standard error; b: the statistical comparison of body weight of the administration group with those in the PBS control group on day 15 of the grouping administration, Two-way ANOVA analysis. | | | | | | |

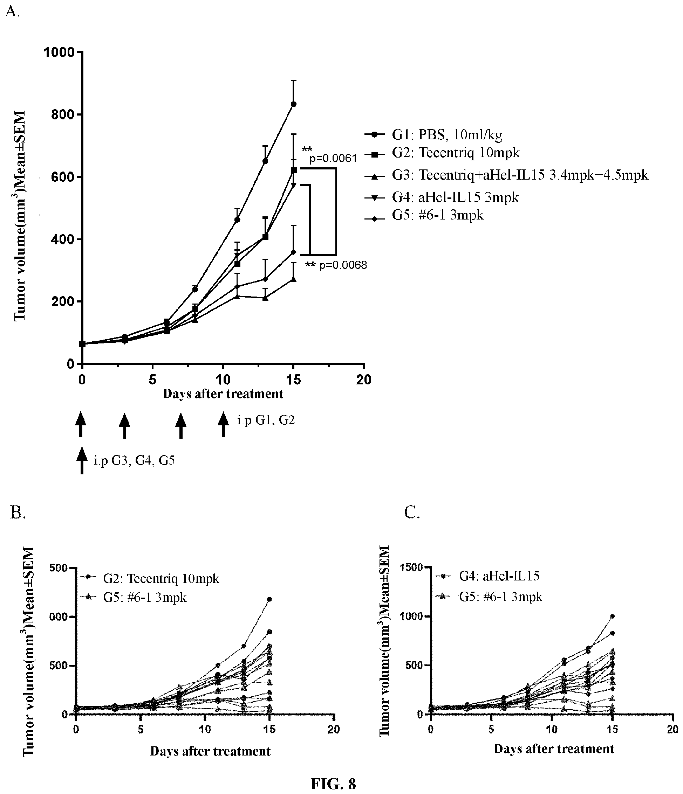

The tumor monitoring results show that the anti-PD-L1-II,15 fusion protein #6-1 had a significant inhibition effect on the growth of A375 tumor subcutaneous xenograft tumor (Table 17 and FIG. 8); compared with the Tecentriq (10 mg/kg) group and the aHel-IL15 (3 mg/kg) group, the #6-1 group (3 mg/kg) had a significantly stronger tumor inhibition effect, showing the efficacy advantage of the fusion protein; the #6-1 group (3 mg/kg) had comparable tumor volume inhibition effect with the Tecentriq + aHel-IL15 (3.4 mg/kg + 4.5 mg/kg) combination group but better safety(Table 18 and FIG. 9).

### Example 11. Detection of Test Drug Content in Tumor Tissue by Enzyme-Linked Immunosorbent Assay (ELISA)

7 × 10⁶ cells/100 µL of human colon cancer cells RKO cells (ATCC, CRL-2577) were subcutaneously inoculated into the right front near the axilla of NPG mice (purchased from Beijing Vitalstar Biotechnology Co., Ltd.). After RKO inoculation and the tumor grew to 300-500 mm³, individuals with too big and too small body weight and tumor volume were removed, and the mice were randomized into 4 groups according to tumor volume. There were a total of 80 mice, with 20 mice in each group. The detailed grouping, administration, and sampling experimental protocols are shown in Table 19 below. After the grouping administration was completed according to the protocol in Table 19, at the scheduled time, blood was collected from the mice, and the mice were euthanized. The intact tumor was taken, washed with PBS, and cross-sectioned. One of the tumor tissues was taken, homogenized, and lysed by adding a lysis buffer (T-PERTMTissue Protein Extraction Reagent: thermo, 78510 plus protease inhibitor mixture: Beyotime, P1046 plus nuclease: millipore, E1014) to give a tissue lysate for ELISA quantitative analysis. Another tissue was taken and cut into small pieces with surgical scissors. A tissue digestive enzyme (Miltenyi, 130-095-929) was added, and the mixture was incubated with shaking for 30 min. After digestion, a stop solution (98% PBS (Hyclone, SH30256) and 2% FBS (Gibco, 10099-141C)) was added, and a single cell suspension was collected by passing through a cell strainer (Thermo, 70 µm\352350\Falcon). 0.5 × 10⁶ single cells were taken for flow cytometry analysis.

ELISA analysis method and results: the drug concentration in the tumor lysate at different time points was measured by enzyme-linked immunosorbent assay (ELISA) to compare the degree and trend of enrichment of the test molecule in the tumor tissue at different doses. The Fc region of the test molecule was captured using an anti-human Fc antibody (Jackson Immuno, #109-006-098). After a standard substance and the test tumor lysate were added, an enzyme-labeled anti-human F(ab)₂ antibody (Jackson Immuno, #109-035-097) bound to the Fab region of the test molecule to determine the concentration of humanized antibodies in the tumor lysate, and the test drug content in the tumor tissue of each individual animal was calculated using the formula below: test drug content in individual tumor (ng) = test drug concentration in the tumor lysate (ng/mL) × volume of the lysate (mL) / weight of the lysed tumor portion (mg) × total tumor weight of the individual (mg).

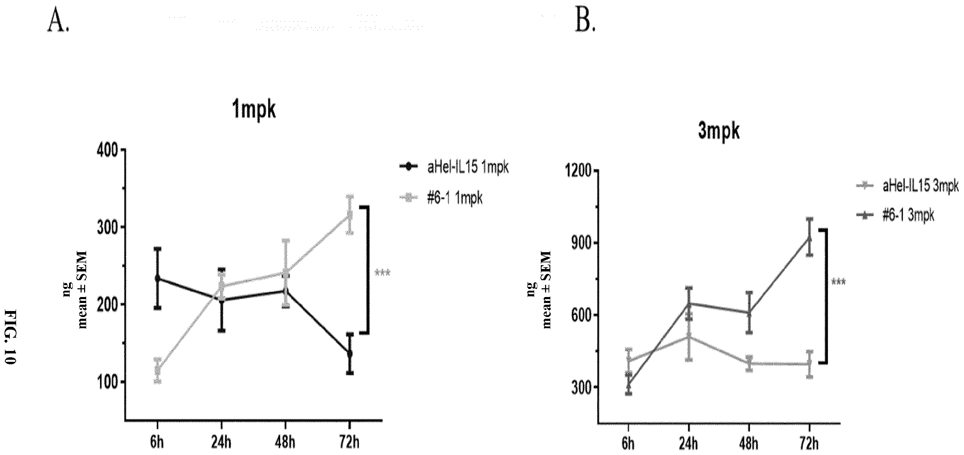

As shown in results of A and B in FIG. 10, compared with the aHel-IL15 isotype control, the #6-1 fusion protein could specifically target PD-L1⁺ tumor cells in the tumor tissue, and generate the drug concentration advantage in the tumor tissue through the targeting effect of the aPD-L1 end, and the concentration increased with the increase of the dose and showed a continuous enrichment trend in 6-72 h. (T test, *P < 0.05, **P < 0.01, ***P < 0.005, ****P < 0.0005)

Flow cytometry method and results: after the single cell suspension was centrifuged, the cells were resuspended with PBS and stained with a live/dead dye (biolegend, 423114) for 20 min. After completion, the cells were washed twice with a staining buffer. A blocking antibody (BD, 55314) was added for 15 min, and a staining antibody CD47 (biolegend, 323124) and PE anti-human IgG Fc (biolegend, 410708) were then added (note: CD47 for labeling the RKO cells, and the anti-human Fc fluorescent antibody can specifically bind to the Fc of the drug). After the staining was completed, flow cytometry was performed. The results show the positive rate of PE anti-human IgG Fc in RKO cells (CD47⁺ cell population).

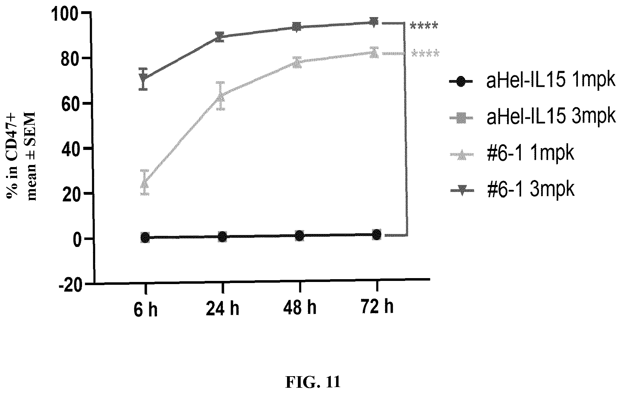

As shown in FIG. 11, the flow cytometry results show that compared with aHel-IL15, the #6-1 fusion protein could specifically bind to tumor cells, with a good dose relationship in high and low doses, and showing a step enrichment trend in 6-72 h. The design goal of the fusion protein was achieved.

The experiments of Examples 6-11 were performed using the fusion protein #6-1-1. The results were confirmed to be exactly the same as the trend of #6-1-2 (i.e., #6-1 as described in Examples 8-11). Similar figures and tables were not repeatedly presented.

**Table 19. Experimental protocol for tissue distribution**

| **Group** | **Test drug** | **Dose (mg/kg)** | **Sampling time point** | **Administration route** | **Frequency** |
|---|---|---|---|---|---|
| 1 | aHel-IL15 | 1 | 6h, 24h, 48h, 72h | Tail vein | Single administration |
| 2 | aHel-IL15 | 3 | 6h, 24h, 48h, 72h | | |
| 3 | #6-1 | 1 | 6h, 24h, 48h, 72h | | |
| 4 | #6-1 | 3 | 6h, 24h, 48h, 72h | | |

In conclusion
Various basic physicochemical parameters such as protein PKa, glycosylation, hydrophilic and hydrophobic properties (determined by sequence and higher structure), isoelectric point, and the like can influence the stability of different formulation formulas.

According to the present application, through screening, a stable formulation formula was selected for the IL-15 fusion protein after mutation. Under accelerated tests of various conditions during investigation, investigation results of parameters such as iCIEF, SE-HPLC, CE-SDS (NR), CE-SDS (R), DLS, insoluble particle, and the like did not change significantly.

## Claims

1. A pharmaceutical composition, wherein the pharmaceutical composition comprises an IL-15 variant fusion protein, a buffer, a protein stabilizer, and a surfactant.

2. The pharmaceutical composition according to claim 1, wherein the IL-15 variant fusion protein comprises the following domains:
(1) an IL-15 variant;
(2) an immunoglobulin molecule fused to the IL-15 variant or a portion thereof;
(3) IL-15Rα.

3. The pharmaceutical composition according to claim 2, wherein the IL-15 variant comprises a mutation at one or more amino acid residues corresponding to Asp8, Ile6, Val3, and His105 of wild-type IL-15.

4. The pharmaceutical composition according to claim 3, wherein the mutation is an amino acid substitution selected from the group consisting of: Asp8Ser (D8S), Asp8Gly (D8G), Asp8 Glu (D8E), Val3Leu (V3L), Ile6Asp (I6D), and/or His105Lys (H105K).

5. The pharmaceutical composition according to any one of claims 1-4, wherein the IL-15 variant comprises the following mutation or combination of mutations: (1) Asp8Ser; (2) Asp8Gly; (3) His105 Lys; (4) Asp8Glu and Val3Leu; (5) Val3Leu and Ile6Asp; or (6) Asp8Ser and His105Lys, preferably Asp8Ser and His105Lys.

6. The pharmaceutical composition according to any one of claims 1-5, wherein the amino acid sequence of the IL-15 variant is set forth in SEQ ID NO: 2, SEQ ID NO: 3, SEQ ID NO: 4, SEQ ID NO: 5, SEQ ID NO: 6, or SEQ ID NO: 7;
preferably, the amino acid sequence of wild-type IL-15 is set forth in SEQ ID NO: 1.

7. The pharmaceutical composition according to any one of claims 1-6, wherein the order in which domains in the IL-15 variant fusion protein are linked from the N-terminus to the C-terminus is:
(1) an immunoglobulin molecule or a portion thereof, IL-15Rα, and an IL-15 variant;
(2) an immunoglobulin molecule or a portion thereof, an IL-15 variant, and IL-15Rα;
(3) an IL-15 variant, IL-15Rα, and an immunoglobulin molecule or a portion thereof;
(4) IL-15Rα, an IL-15 variant, and an immunoglobulin molecule or a portion thereof;
preferably, when the IL-15Rα or the IL-15 variant is fused to the immunoglobulin molecule, they are fused at the N-terminus of the heavy chain variable region of the immunoglobulin molecule or the C-terminus of the immunoglobulin Fc region; when the IL-15Rα or the IL-15 variant is fused to the immunoglobulin Fc region, they are fused at the N-terminus or C-terminus of the immunoglobulin Fc region.

8. The pharmaceutical composition according to any one of claims 1-7, wherein the IL-15 variant is fused to an immunoglobulin molecule or a portion thereof with or without a linker peptide, or the IL-15 variant is fused to IL-15Rα with or without a linker peptide; preferably, a linker peptide is used; preferably, the linker peptide set forth in SEQ ID NO: 13, SEQ ID NO: 14, SEQ ID NO: 15, or SEQ ID NO: 16 is used;
preferably, the IL-15 variant is fused to IL-15Rα with or without a linker peptide, and is then fused to the immunoglobulin molecule or the portion thereof; preferably, a linker peptide is used; preferably, the linker peptide set forth in SEQ ID NO: 13, SEQ ID NO: 15, or SEQ ID NO: 16 is used.

9. The pharmaceutical composition according to any one of claims 1-8, wherein the immunoglobulin molecule is an anti-PD-L1 antibody or antigen-binding fragment;
preferably, the anti-PD-L1 antibody or antigen-binding fragment is selected from Tecentriq, KN-035, or 794-h1-71; more preferably, the heavy chain of the anti-PD-L1 antibody has the sequence set forth in SEQ ID NO: 19 or SEQ ID NO: 20, and the light chain of the anti-PD-L1 antibody has the sequence set forth in SEQ ID NO: 21;

10. The pharmaceutical composition according to any one of claims 1-8, wherein the immunoglobulin molecule portion is an immunoglobulin Fc region;
preferably, the immunoglobulin Fc region is selected from the Fc region of any one of IgG1, IgG2, IgG3, IgG4, IgA, IgM, IgE, or IgD, preferably comprising a sequence of the constant region of human or murine antibody IgG1, IgG2, IgG3, or IgG4;
more preferably, the amino acid sequence of the immunoglobulin Fc region is set forth in SEQ ID NO: 12.

11. The pharmaceutical composition according to any one of claims 1-10, wherein the IL-15 variant fusion protein comprises:
(1) monomer one independently having the sequence set forth in SEQ ID NO: 22, 23, 24, 25, or 26, and comprising the heavy chain of the anti-PD-L1 antibody; the light chain of the anti-PD-L1 antibody having the sequence set forth in SEQ ID NO: 21;
(2) an amino acid sequence having at least 90% identity, preferably at least 91%, 92%, 93%, 94%, 95%, 96%, 97%, 98%, or 99% identity, to the sequence set forth in (1) above.

12. The pharmaceutical composition according to any one of claims 1-11, wherein the concentration of the IL-15 variant fusion protein is 1 mg/mL to 100 mg/mL, preferably 25-95 mg/mL, and more preferably 50 mg/mL or 25 mg/mL.

13. The pharmaceutical composition according to any one of claims 1-12, wherein the buffer is selected from an acetic acid-sodium acetate buffer, a citric acid-sodium citrate buffer, a histidine-histidine hydrochloride buffer, and a disodium hydrogen phosphate-sodium dihydrogen phosphate buffer, preferably a histidine-histidine hydrochloride buffer.

14. The pharmaceutical composition according to claim 13, wherein the concentration of the buffer is 10-100 mM, preferably 15-30 mM, and more preferably 20 mM.

15. The pharmaceutical composition according to any one of claims 1-14, wherein the protein stabilizer is selected from sodium chloride, glycine, arginine hydrochloride, sucrose, trehalose, mannitol, and sorbitol, preferably sucrose and trehalose.

16. The pharmaceutical composition according to claim 15, wherein the concentration of the protein stabilizer is 1-10% (w/v), preferably 2% (w/v), 4.5% (w/v), or 8% (w/v), and more preferably 8% (w/v).

17. The pharmaceutical composition according to any one of claims 1-16, wherein the surfactant is selected from polysorbate, poloxamer, and glycerol fatty acid ester, preferably poloxamer 188.

18. The pharmaceutical composition according to claim 17, wherein the concentration of the surfactant is 0.01-0.1% (w/v), preferably 0.02% (w/v), 0.04% (w/v), 0.06% (w/v), or 0.08% (w/v).

19. The pharmaceutical composition according to any one of claims 1-18, wherein the pH of the pharmaceutical composition is about 4.5-7.5, preferably 4.5-6.5, and more preferably 6.

20. The pharmaceutical composition according to any one of claims 1-19, wherein the pharmaceutical composition comprises:
a) the IL-15 variant fusion protein at a concentration of 1 mg/mL to 100 mg/mL,
b) a histidine-histidine hydrochloride buffer at a concentration of 10-100 mM, pH 4.5-7.5,
c) trehalose at a concentration of 1-10% (w/v), and
d) poloxamer at a concentration of 0.01-0.1% (w/v);
preferably, the pharmaceutical composition comprises:
a) the IL-15 variant fusion protein at a concentration of 25 mg/mL to 95 mg/mL,
b) a histidine-histidine hydrochloride buffer at a concentration of 15-30 mM, pH 5.5-6.5,
c) trehalose at a concentration of 2-8% (w/v), and
d) poloxamer 188 at a concentration of 0.04% (w/v);
preferably, the pharmaceutical composition is in a liquid form or a freeze-dried powder formulation.

21. A method for preparing the pharmaceutical composition according to any one of claims 1-20, wherein the method comprises the step of performing buffer exchange on an IL-15 variant fusion protein solution, the buffer is preferably a histidine-histidine hydrochloride buffer, the concentration of the buffer is preferably 20 mM, and the pH of the buffer is preferably 6;
preferably, the preparation method further comprises the step of freeze-drying;
more preferably, the freeze-drying step comprises: (1) cooling the drying box of a vacuum freeze dryer to -45 °C and keeping for 3-5 h; (2) heating the drying box to -5 °C and keeping for 3-5 h; (3) cooling the drying box to -45 °C and keeping for 3-5 h (in the pre-freezing process of steps (1) - (3), the heating rate and the cooling rate are kept at 0.5-1 °C/min); (4) the degree of vacuum in primary drying is 0.1 mBar, the temperature is kept at -30 °C to 20 °C, and the total time of the step should be greater than 30 h; (5) after primary drying, heating to 25 °C at a heating rate of 0.1-0.3 °C/min, starting desorption drying, keeping the degree of vacuum at 0.1 mBar for 1-2 h, then creating ultimate vacuum, and keeping the temperature unchanged for longer than 15 h.

22. A pharmaceutical composition comprising an IL-15 variant fusion protein, wherein the pharmaceutical composition is prepared by the method according to claim 21.

23. A freeze-dried formulation comprising an IL-15 variant fusion protein, wherein the formulation is obtained by freeze-drying of the pharmaceutical composition according to any one of claims 1 to 20.

24. A reconstituted solution comprising an IL-15 variant fusion protein, wherein the reconstituted solution is prepared by reconstituting the freeze-dried formulation according to claim 23.

25. Use of the pharmaceutical composition according to any one of claims 1-20, a product prepared by the method according to claim 21, the pharmaceutical composition according to claim 22, the freeze-dried formulation according to claim 23, or the reconstituted solution according to claim 24 in preparing a drug for preventing and/or treating a disease in an individual, wherein the disease is preferably a tumor or an inflammatory disease;
preferably, the tumor is selected from glioblastoma, prostate cancer, hematologic cancer, B-cell tumor, multiple myeloma, B-cell lymphoma, B-cell non-Hodgkin's lymphoma, Hodgkin's lymphoma, chronic lymphocytic leukemia, acute myelogenous leukemia, cutaneous T-cell lymphoma, T-cell lymphoma, solid tumor, urothelial/bladder cancer, melanoma, lung cancer, renal cell carcinoma, breast cancer, gastric and esophageal cancer, prostate cancer, pancreatic cancer, colorectal cancer, ovarian cancer, non-small cell lung cancer, and head and neck squamous cell carcinoma.

26. A method for preventing and/or treating a disease in an individual, wherein the method comprises administering to a patient in need thereof the pharmaceutical composition according to any one of claims 1-20, a product prepared by the method according to claim 21, the pharmaceutical composition according to claim 22, the freeze-dried formulation according to claim 23, or the reconstituted solution according to claim 24, wherein the disease is preferably a tumor or an inflammatory disease;
preferably, the tumor is selected from glioblastoma, prostate cancer, hematologic cancer, B-cell tumor, multiple myeloma, B-cell lymphoma, B-cell non-Hodgkin's lymphoma, Hodgkin's lymphoma, chronic lymphocytic leukemia, acute myelogenous leukemia, cutaneous T-cell lymphoma, T-cell lymphoma, solid tumor, urothelial/bladder cancer, melanoma, lung cancer, renal cell carcinoma, breast cancer, gastric and esophageal cancer, prostate cancer, pancreatic cancer, colorectal cancer, ovarian cancer, non-small cell lung cancer, and head and neck squamous cell carcinoma.
